# EUROPEAN PATENT SPECIFICATION

(11) **EP 1 924 287 B1**
(45) Date of publication and mention of the grant of the patent: **14.01.2015**
(21) Application number: 06801876.1
(22) Date of filing: 18.08.2006
(51) Int. Cl.: A61K 39/395, A61P 25/24

(54) **METHOD OF TREATING DEPRESSION USING A TNF-ALPHA ANTIBODY**
VERFAHREN ZUR BEHANDLUNG VON DEPRESSIONEN MIT EINEM TNF-ALPHA-ANTIKÖRPER
PROCÉDÉ DE TRAITEMENT DE LA DÉPRESSION AU MOYEN D'UN ANTICORPS ANTI-TNF-ALPHA

(30) Priority: 19.08.2005 US 709998 P
(43) Date of publication of application: 28.05.2008
(73) Proprietor: AbbVie Biotechnology Ltd, HM 11 Hamilton (BM)
(72) Inventor: HOFFMAN, Rebecca, S, Wilmette, IL 60091 (US); DECKER, Michael, W., Mundelein, IL 60060 (US); BASSO, Ana, M, Libertyville, IL 60048 (US); RUETER, Lynne, E, Round Lake Beach, IL 60073 (US); ABI-SAAB, Walid, M, Lake Forest, IL 60045 (US)
(74) Representative: Modiano, Micaela Nadia
(86) International application number: PCT/US2006/032365
(87) International publication number: WO 2007/024705

(56) References cited:
- WO-A2-02/12502
- US-A1- 2003 049 256
- US-A1- 2005 095 246
- US-B1- 6 509 015
- BRAMBILLA F ET AL: "Blood levels of cytokines in elderly patients with major depressive disorder.", ACTA PSYCHIATRICA SCANDINAVICA APR 1998, vol. 97, no. 4, April 1998 (1998-04), pages 309-313, ISSN: 0001-690X
- KAGAYA A ET AL: "Plasma concentrations of interleukin-1beta, interleukin-6, soluble interleukin-2 receptor and tumor necrosis factor alpha of depressed patients in Japan.", NEUROPSYCHOBIOLOGY 2001, vol. 43, no. 2, 2001, pages 59-62, ISSN: 0302-282X
- LEO ROBERTO ET AL: "Association between enhanced soluble CD40 ligand and proinflammatory and prothrombotic states in major depressive disorder: pilot observations on the effects of selective serotonin reuptake inhibitor therapy.", THE JOURNAL OF CLINICAL PSYCHIATRY NOV 2006, vol. 67, no. 11, November 2006 (2006-11), pages 1760-1766, ISSN: 1555-2101
- MIKOVA O ET AL: "Increased serum tumor necrosis factor alpha concentrations in major depression and multiple sclerosis", EUROPEAN NEUROPSYCHOPHARMACOLOGY, vol. 11, no. 3, June 2001 (2001-06), pages 203-208, ISSN: 0924-977X
- O'BRIEN SINEAD M ET AL: "Plasma cytokine profiles in depressed patients who fail to respond to selective serotonin reuptake inhibitor therapy.", JOURNAL OF PSYCHIATRIC RESEARCH 2007 APR-JUN, vol. 41, no. 3-4, April 2007 (2007-04), pages 326-331, ISSN: 0022-3956
- PAVÓN LENIN ET AL: "Th2 cytokine response in Major Depressive Disorder patients before treatment.", JOURNAL OF NEUROIMMUNOLOGY MAR 2006, vol. 172, no. 1-2, March 2006 (2006-03), pages 156-165, ISSN: 0165-5728
- SIMON N M ET AL: "A detailed examination of cytokine abnormalities in Major Depressive Disorder.", EUROPEAN NEUROPSYCHOPHARMACOLOGY : THE JOURNAL OF THE EUROPEAN COLLEGE OF NEUROPSYCHOPHARMACOLOGY MAR 2008, vol. 18, no. 3, March 2008 (2008-03), pages 230-233, ISSN: 0924-977X
- YANG KUN ET AL: "Levels of serum interleukin (IL)-6, IL-1beta, tumour necrosis factor-alpha and leptin and their correlation in depression.", THE AUSTRALIAN AND NEW ZEALAND JOURNAL OF PSYCHIATRY MAR 2007, vol. 41, no. 3, March 2007 (2007-03), pages 266-273, ISSN: 0004-8674
- TYRING S ET AL: "Etanercept and clinical outcomes, fatigue, and depression in psoriasis: double-blind placebo-controlled randomised phase III trial", THE LANCET, LANCET LIMITED. LONDON, GB, vol. 367, no. 9504, 7 January 2006 (2006-01-07), pages 29-35, XP027947305, ISSN: 0140-6736 [retrieved on 2006-01-07]

## Description

### BACKGROUND OF THE INVENTION

Depression, including major depression, effects approximately 20-25% of women and 7-12% of men in Western countries at some point in their lifetime.

Depression is the most common mental disease and the fourth most important cause of disability worldwide. It is expected that rates of depression in the population will increase in the future. Many patients remain undiagnosed and undertreated due to social stigma associated with psychiatric treatments, inappropriate training of general practitioners for the diagnosis of the disease, or low awareness between patients and doctors of depression as a treatable illness.

Hypersecretion of pro-inflammatory cytokines such as TNF-α, IL-1β, and IL-6, has been reported in depressed patients, suggesting that cytokine-mediated pathways could be involved in the etiopathogenesis of depression (Levine, J. et al. Neuropsychobiology 40, 171-6 (1999); Sluzewska, A. et a/. Indicators of Immune activation has been identified in major depression. Psychiatry Res 64, 161-7 (1996)). Patients with major depression have higher levels of TNF-α, C-reactive protein (CRP) and leukocyte count than control patients (Tuglu et al. Psychopharmacology (Berl) 170, 429-33 (2003)). Two independent clinical studies by Penninx et al. (Biol Psychiatry, 54, 566-72 (2003)) and Trzonkowski et al. (Brain Behav Immun 18, 135-48 (2004)) also reported an association between high levels of inflammatory markers (TNF-α, IL-6 and CRP) and depressed mood in aged patients, suggesting that depressed mood causes and/or is caused by systemic inflammation (Pennix, *supra* and Trzonkowski *supra*). Increased serum TNF-α concentrations have also been associated with both major depression disorder and multiple sclerosis (Mikova et al. Eur Neuropsychopharmacol 11, 203-8 (2001)). Increased levels of cytokines in depressed patients can be normalized after chronic antidepressant treatment with serotonin re-uptake inhibitors (SSRIs) (Tuglu et al. Psychopharmacology (Berl) 170, 429-33 (2003)).

Despite different treatments for depression there are still several unmet needs and room from improvements for medications including improved efficacy, better tolerability, rapid onset of action and prevention of relapse and recurrence of depressive episodes. Current drug therapies are effective in only 50-70% of patients. Among responders, about 50% do not achive full remission, 55-60% of patients experience recurrence within 5 years of the treatment and 80% suffer a recurrence within 15 years. Important progress in the treatment of affective disorders has been achieved since the serendipitous finding of monoamine oxidase inhibitors MAOi (isoniazid and iproniazid) originally developed for the treatment of tuberculosis in 1951, the discovery of tricyclics antidepressants in the 1960s, and more recently the SSRIs or other compounds with a less defined pharmacology. Current antidepressant drugs are mainly based on the monoamine hypothesis of depression. SSRIs represent the first line of treatment. However, although these compounds are safer and with less side effect than other antidepressants, little improvement in terms of efficacy, onset of action or prevention of relapse has been observed.

US 2003/049256 A1 discloses methods for treating neurological or neuropsychiatric diseases or disorders in humans by administering to the human a therapeutically effective dose of specific biologies. The biologies of consideration include antagonists of tumor necrosis factor or interleukin-1. The administration of these biologies is performed by specific methods, most, but not all of which fall into the category of anatomically localized administration designed for perispinal use. Anatomically localized administration involving perispinal use includes, but is not limited to the subcutaneous, intramuscular, interspinous, epidural, peridural, parenteral or intrathecal routes.

US 2005/095246 A2 discloses methods and devices to attenuate tumor necrosis factor (TNF) and other pro-inflammatory mediators in the CNS to treat neurological, neurodegenerative, neuropsychiatric disorders, pain and brain injury are described. More particularly, TNF blocking agents that target intracellular signals and downstream effects associated with the production and secretion on TNF are described. Devices described include therapy delivery devices comprising a reservoir capable of housing a TNF blocking agent and a catheter operably coupled to the device and adapted to deliver the TNF blocking agent to a target site within a subject.

### SUMMARY OF THE INVENTION

There is a need for an effective and safe method for treating depression. In addition, there is a need for a depression therapy which is both effective and has few side effects. The invention provides an isolated anti-TNFα antibody or an antigen-binding portion thereof for use in the treatment of depression based on the inhibition of peripheral cytokine activity, especially TNFα

The instant invention therefore provides an isolated human anti-TNFα antibody, or an antigen binding portion thereof, for use in the treatment of depression, wherein the human anti-TNFα antibody, or the antigen binding portion thereof, is subcutaneously administered to the subject and wherein the subcutaneous administration excludes perispinal administration, such that peripheral TNFα activity is inhibited.

In one embodiment, the TNFα inhibitor for use in the invention is etanercept. In another embodiment, the TNFα inhibitor for use in the invention is a TNFα antibody, including infliximab, adalimumab, and golimumab.

The invention provides an isolated human anti-TNFα antibody, or an antigen binding portion thereof, for use in inhibiting TNFα activity in a subject suffering from depression by subcutaneously administering the same to the subject and wherein the subcutaneous administration excludes perispinal administration, such that depression is treated. The invention also provides. an isolated human anti-TNFα antibody, or an antigen binding portion thereof, for use in improving the mood of a subject having depression comprising subcutaneously administering to the subject an isolated human anti-TNFα antibody, or an antigen binding portion thereof, wherein the subcutaneous administration excludes perispinal administration, such that the mood of the subject having depression is improved.

The invention describes an isolated human anti-TNFα antibody, or an antigen binding portion thereof, for use in a method for treating depression in a subject having an increased level of serum TNFα comprising subcutaneously administering to the subject an isolated human anti-TNFα antibody, or an antigen binding portion thereof, wherein the subcutaneous administration excludes perispinal administration, such that the serum level of TNFα is decreased relative to pre-treatment levels. Another aspect of the invention is an isolated human anti-TNFα antibody, or an antigen binding portion thereof, for use in a method of inhibiting peripheral TNFα activity in a subject suffering from depression comprising subcutaneously administering an TNFα antibody to said subject, wherein the subcutaneous administration excludes perispinal administration such that peripheral TNFα activity is inhibited. The invention also includes an isolated human anti-TNFα antibody, or an antigen binding portion thereof, for use in a method for treating TNFα-mediated depression in a subject suffering from said depression comprising subcutaneously administering to the subject an isolated human anti-TNFα antibody, or an antigen binding portion thereof, wherein the subcutaneous administration excludes perispinal administration, such that the depression is treated. The instant disclosure also includes a method of achieving a HAM-D score of ≤ 7 in a subject having depression comprising systemically administering to the subject a TNFα antibody, or an antigen-binding portion thereof, such that the subject's HAM-D score is ≤ 7.

In one embodiment, the TNFα antibody, or an antigen-binding portion thereof, is selected from the group consisting of infliximab, golimumab, and adalimumab.

In one embodiment, the TNFα antibody, or an antigen-binding portion thereof, is a human antibody, or antigen-binding portion thereof.

In one embodiment, the human antibody, or antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an TC₅₀ of 1 x 10⁻⁷ M or less.

In one embodiment, the human antibody, or antigen-binding portion thereof, is D2B7 or adalimumab.

In one embodiment, the human TNFα antibody, or antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

In another embodiment, the human TNFα antibody, or antigen-binding portion thereof, has the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1,3,4,6, 7, 8 and/or 9;
c) has a heavy chain CDR3 domain comprising the amino acid sequence of SEQ ID NO: 4, or modified from SEQ ID NO: 4 by a single alanine substitution at position 2,3,4,5,6,8,9,10 or 11 or by one to five conservative amino acid substitutions at positions 2, 3, 4, 5, 6, 8, 9, 10, 11 and/or 12.

In still another embodiment, the human TNFα antibody, or antigen-binding portion thereof, comprises a light chain variable region (LCVR) comprising the amino acid sequence of SEQ ID NO:1 and a heavy chain variable region (HCVR) comprising the amino acid sequence of SEQ ID NO: 2. In yet another embodiment, the human TNFα antibody, or antigen-binding portion thereof, is D2E7.

The isolated human anti-TNFα antibody, or an antigen binding portion thereof of the invention is for use in treating major depression. In one embodiment, the major depression is a single episode. In another embodiment, the major depression is recurrent. In another embodiment, the major depression is refractory or treatment resistant depression.

The isolated human anti-TNFα antibody, or an antigen binding portion thereof of the invention may also be for use in treating depression which is a cyclothymic disorder. The isolated human anti-TNFα antibody, or an antigen binding portion thereof of the invention may also be for use in treating depression selected from the group consisting of dysthmic disorder, bipolar disorder I, and bipolar disorder II. In one embodiment, the disorder occurs in combination with catatonic features, melancholic features, or with atypical features of postpartum depression.

The human TNFα antibody or antigen binding portion thereof according to the invention is for use in subcutaneously administering to the subject, wherein the subcutaneous administration excludes perispinal administration.

In an embodiment, the subcutaneous administration of the human TNFα antibody, or antigen-binding portion thereof, is peripheral.

In one embodiment of the invention, the subject has an additional disorder associated with increased levels of TNFα. In one embodiment of the invention, the subject has an additional disorder which is a TNFα related disorder. In another embodiment, the subject has an additional disorder selected from the group consisting of coronary heart disease, a neurodegenerative disease, an autoimmune disease, an intestinal disorder, and an infectious disease. In one embodiment, the neurodegenerative disease is stroke. In another embodiment, the autoimmune disorder is selected from the group consisting of psoriasis, psoriatic arthritis, and rheumatoid arthritis. In still another embodiment, the subject further has a disorder selected from the group consisting of Behcet's disease, asthma, and Niemann-Pick disease. In one embodiment, the intestinal disorder is inflammatory bowel disease or Crohn's disease.

In one embodiment, the invention includes further administering an antidepressant agent to the subject in combination with a human TNFα, antibody, or antigen-binding portion thereof.

In still another embodiment, the human TNFα antibody, or antigen-binding portion thereof, is for use in administration on a biweekly dosing regimen. In another embodiment, the antibody is for use in admi nistration on dosing regimen selected from the group consisting of a biweekly dosing regimen, a multiple variable dose regimen, and a weekly dosing regimen. In yet another embodiment, the human TNFα antibody, or antigen-binding portion thereof, is for use in administration as a 40 mg dose. The instant disclosure also provides kits containing a human TNFα antibody, or antigen-binding portion thereof, and instructions for administering the antibody to an affect The instant disclosure provides a kit comprising a packaging material; a TNFα antibody, or antigen-binding portion thereof; and a label or package insert contained within the packaging material indicating that the TNFα antibody, or antigen-binding portion thereof, may be used for the treatment of depression.

The package insert further contains instructions for subcutaneous administration, excluding perispinal administration of the TNFα antibody, or antigen-binding portion thereof.

In one embodiment, the TNFα antibody, or antigen-binding portion thereof, comprises a dose of about 40 mg.

In one embodiment, the TNFα antibody, or antigen-binding portion thereof, is selected from the group consisting of adalimumab, infliximab, and golimumab.

In one embodiment, the human antibody, or an antigen-binding portion thereof, dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

### DETAILED DESCRIPTION OF THE INVENTION

### I. Definitions

In order that the present invention may be more readily understood, certain terms are first defined.

The term "human TNFα" (abbreviated herein as hTNFα, or simply hTNF), as used herein, is intended to refer to a human cytokine that exists as a 17 kD secreted form and a 26 kD membrane associated form, the biologically active form of which is composed of a trimer of noncovalently bound 17 kD molecules. The structure of hTNFα is described further in, for example, Pennica, D., et al. (1984) Nature 312:724-729; Davis, J.M., et al. (1987) Biochemistry 26:1322-1326; and Jones, E.Y., et al. (1989) Nature 338:225-228. The term human TNFα is intended to include recombinant human TNFα (rhTNFα), which can be prepared by standard recombinant expression methods or purchased commercially (R & D Systems, Catalog No. 210-TA, Minneapolis, MN). TNFα is also referred to as TNF.

As used herein, the term "TNFα inhibitor" refers to an agent which interferes with tumor necrosis factor alpha (TNFα) activity. The term also includes each of the anti-TNFα human antibodies and antibody portions described in U.S. Patent Nos. 6,090,382; 6,258,562; 6,509,015, and in U.S. Patent Application Serial Nos. 09/801185 and 10/302356. In one embodiment, the TNFα inhibitor used in the invention is an anti-TNFα antibody, or a fragment thereof, including infliximab (Remicade^{®}, Johnson and Johnson; described in U.S. Patent No. 5,656,272, CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (Humira^{®} Abbott Laboratories, a human anti-TNF mAb, described in US 6,090,382 as D2E7). Additional TNF antibodies which may be used in the invention are described in U.S. Patent Nos. 6,593,458; 6,498,237; 6,451,983; and 6,448,380. In another embodiment, the TNFα inhibitor is a TNF fusion protein, *e.g.*, etanercept (Enbrel^{®}, Amgen; described in WO 91/03553 and WO 09/406476, or p55TNFR1gG (Lenercept). In another embodiment, the TNFα inhibitor is a recombinant TNF binding protein (r-TBP-I) (Serono).

The term "antibody", as used herein, is intended to refer to immunoglobulin molecules comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The antibodies of the invention are described in further detail in U.S. Patent Nos. 6,090,382; 6,258,562; and 6,509,015, and in U.S. Patent Application Serial Nos. 09/801185 and 10/302356.

The term "antigen-binding portion" of an antibody (or simply "antibody portion"), as used herein, refers to one or more fragments of an antibody that retain the ability to specifically bind to an antigen (*e.g.*, hTNFα). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.*, Bird et al. (1988) Science 242:423-426; and Huston et al. (1988) Proc. Natl. Acad Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R.J., et al. (1994) Structure 2:1121-1123). The antibody portions of the invention are described in further detail in U.S. Patent Nos. 6,090,382, 6,258,562, 6,509,015, and in U.S. Patent Application Serial Nos. 09/801185 and 10/302356.

Binding fragments are produced by recombinant DNA techniques, or by enzymatic or chemical cleavage of intact immunoglobulins. Binding fragments include Fab, Fab', F(ab')₂, Fabc, Fv, single chains, and single-chain antibodies. Other than "bispecific" or "bifunctional" immunoglobulins or antibodies, an immunoglobulin or antibody is understood to have each of its binding sites identical. A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, *e.g.,* Songsivilai & Lachmann, Clin. Exp. Immunol. 79:315-321 (1990); Kostelny et al., J. Immunol. 148, 1547-1553 (1992).

A "conservative amino acid substitution", as used herein, is one in which one amino acid residue is replaced with another amino acid residue having a similar side chain. Families of amino acid residues having similar side chains have been defined in the art, including basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.*, threonine, valine, isoleucine) and aromatic side chains (*e.g.,* tyrosine, phenylalanine, tryptophan, histidine).

The term "human antibody", as used herein, is intended to include antibodies having variable and constant regions derived from human germline immunoglobulin sequences. The human antibodies of the invention may include amino acid residues not encoded by human Germline immunoglobulin sequences (*e.g*., mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences. In one embodiment, the human TNF antibody is adalimumab (also referred to as Humira and D2E7).

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (*e.g.*, a mouse) that is transgenic for human immunoglobulin genes (see *e.g.*, Taylor, L.D. et al. (1992) Nucl. Acids Res. 20:6287) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo*.

An "isolated antibody", as used herein, is intended to refer to an antibody that is substantially free of other antibodies having different antigenic specificities (*e.g.*, an isolated antibody that specifically binds hTNFα is substantially free of antibodies that specifically_bind antigens other than hTNFα). An isolated antibody that specifically binds hTNFα may, however, have cross-reactivity to other antigens, such as TNFα molecules from other species (discussed in further detail below). Moreover, an isolated antibody may be substantially free of other cellular material and/or chemicals.

A "neutralizing antibody", as used herein (or an "antibody that neutralized hTNFα activity"), is intended to refer to an antibody whose binding to hTNFα results in inhibition of the biological activity of hTNFα. This. inhibition of the biological activity of hTNFα can be assessed by measuring one or more indicators of hTNFα biological activity, such as hTNFα-induced cytotoxicity (either *in vitro* or *in vivo*), hTNFα-induced cellular activation and hTNFα binding to hTNFα receptors. These indicators of hTNFα biological activity can be assessed by one or more of several standard *in vitro* or *in vivo* assays known in the art (see U.S. Patent No. 6,090,382). Preferably, the ability of an antibody to neutralize hTNFα activity is assessed by inhibition of hTNFα-induced cytotoxicity of L929 cells. As an additional or alternative parameter of hTNFα activity, the ability of an antibody to inhibit hTNFα-induced expression of ELAM-1 on HUVEC, as a measure of hTNFα-induced cellular activation, can be assessed.

The term "surface plasmon resonance", as used herein, refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, NJ). For further descriptions, see Example 1 of U.S. Patent 6,258,562 and Jönsson et al. (1993) Ann. Biol. Clin. 51:19; Jönsson et al. (1991) Biotechniques 11:620-627; Johnsson et al. (1995) J. Mol. Recognit. 8:125; and Johnnson et al. (1991) Anal. Biochem.198:268.

The term "K_{off}", as used herein, is intended to refer to the off rate constant for dissociation of an antibody from the antibody/antigen complex.

The term "K_{d}", as used herein, is intended to refer to the dissociation constant of a particular antibody-antigen interaction.

The term "IC₅₀" as used herein, is intended to refer to the concentration of the inhibitor required to inhibit the biological endpoint of interest, *e.g.*, neutralize cytotoxicity activity.

The term "nucleic acid molecule", as used herein, is intended to include DNA molecules and RNA molecules. A nucleic acid molecule may be single-stranded or double-stranded, but preferably is double-stranded DNA.

The term "isolated nucleic acid molecule", as used herein in reference to nucleic acids encoding antibodies or antibody portions (*e.g*., VH, VL, CDR3) that bind hTNFα, is intended to refer to a nucleic acid molecule in which the nucleotide sequences encoding the antibody or antibody portion are free of other nucleotide sequences encoding antibodies or antibody portions that bind antigens other than hTNFα, which other sequences may naturally flank the nucleic acid in human genomic DNA. Thus, for example, an isolated nucleic acid of the invention encoding a VH region of an anti-hTNFα antibody contains no other sequences encoding other VH regions that bind antigens other than hTNFα.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The term "dose," as used herein, refers to an amount of TNFα inhibitor, e.g., a TNFα antibody, which is administered to a subject.

The term "multiple-variable dose" includes different doses of a TNFα inhibitor which are administered to a subject for therapeutic treatment. "Multiple-variable dose regimen" or "multiple-variable dose therapy" describe a treatment schedule which is based on administering different amounts of a human TNFα antibody, or antigen-binding portion thereof, at various time points throughout the course of treatment. In one embodiment, the invention describes a multiple-variable dose method of treatment comprising an induction phase and a treatment phase, wherein a human TNFα antibody, or antigen-binding portion thereof, is administered at a higher dose during the induction phase than the treatment phase. Multiple-variable dose regimens using the human TNFα antibody of the invention are described in U.S. Appln. No. 11/104117.

In reference to a multiple variable dose, the term "induction phase" or "loading phase", refers to a period of treatment comprising administration of a TNFα inhibitor to a subject in order to attain a threshold level. During the induction phase, at least one induction dose of TNFα inhibitor is administered to a subject suffering from a disorder in which TNFα is detrimental. The term "threshold level", as used herein, refers to a therapeutically effective level of a TNFα inhibitor in a subject A threshold level is achieved by administering at least one induction dose during the induction phase of treatment. Any number of induction doses may be administered to achieve a threshold level of a human TNFα antibody, or antigen-binding portion thereof. Once a threshold level is achieved, the treatment phase is initiated.

The term "induction dose" or "loading dose," used interchangeably herein, refers to the first dose of a human TNFα antibody, or antigen-binding portion thereof, which is larger in comparison to the maintenance or treatment dose. The induction dose can be a single dose or, alternatively, a set of doses. The induction dose is often used to bring the drug in the body to a steady state amount, and may be used to which to achieve maintenance drug levels quickly. An induction dose is subsequently followed by administration of smaller doses of a human TNFα antibody, or antigen-binding portion thereof, *i.e.*, the treatment dose. The induction dose is administered during the induction phase of therapy. In one embodiment of the invention, the induction dose is at least twice the given amount of the treatment dose. In another embodiment of the invention, the induction dose of D2E7 is about 160 mg. In another embodiment, the induction dose of D2E7 is about 80 mg.

The term "treatment phase" or "maintenance phase", as used herein, refers to a period of treatment comprising administration of a human TNFα antibody, or antigen-binding portion thereof, to a subject in order to maintain a desired therapeutic effect. The treatment phase follows the induction phase, and, therefore, is initiated once a threshold level is achieved.

The term "treatment dose" or "maintenance dose" is the amount of a human TNFα antibody, or antigen-binding portion thereof, or taken by a subject to maintain or continue a desired therapeutic effect. A treatment dose is administered subsequent to the induction dose. A treatment dose can be a single dose or, alternatively, a set of doses. A treatment dose is administered during the treatment phase of therapy. Treatment doses are smaller than the induction dose and can be equal to each other when administered in succession. In one embodiment, the invention describes at least one induction dose of D2E7 of about 160 mg, followed by at least one treatment dose of about 80 mg. In another embodiment, the invention describes at least one induction dose of D2E7 of 80 mg, followed by at least one treatment dose of 40 mg. In still another embodiment, the treatment dose is administered at least two weeks following the induction dose.

A "dosage regimen" or "dosing regimen" includes a treatment regimen based on a determined set of doses. In one embodiment, the TNFα antibody is administered for the treatment of depression using dosing regimen selected from the group consisting of a biweekly dosing regimen, a multiple variable dose regimen, and a weekly dosing regimen In one embodiment, the invention describes a multiple variable dosage regimen for the treatment of depression, wherein a TNFα antibody, such as adalimumab/D2E7, is first administered as an induction dose and then administered in treatment doses which are lower than that of the induction dose.

The term "dosing", as used herein, refers to the administration of a substance (*e.g.*, a human TNFα antibody, or antigen-binding portion thereof) to achieve a therapeutic objective (*e.g.*, the treatment of a TNFα-associated disorder).

The terms "biweekly dosing regimen", "biweekly dosing", and "biweekly administration", as used herein, refer to the time course of administering a substance (*e.g.*, an anti-TNFα antibody) to a subject to achieve a therapeutic objective (*e.g.*, the treatment of a TNFα-associated disorder). The biweekly dosing regimen is not intended to include a weekly dosing regimen. Preferably, the substance is administered every 9-19 days, more preferably, every 11-17 days, even more preferably, every 13-15 days, and most preferably, every 14 days. Biweekly dosing regimens which may be used in accordance with the invention are described in US application no. 10/163657.

The term "combination" as in the phrase "a first agent in combination with a second agent" includes co-administration of a first agent and a second agent, which for example may be dissolved or intermixed in the same pharmaceutically acceptable carrier, or administration of a first agent, followed by the second agent, or administration of the second agent, followed by the first agent. The present invention, therefore, includes methods of combination therapeutic treatment and combination pharmaceutical compositions. In one embodiment, the invention provides a combination therapy for treating depression or symptoms related thereto comprising administering a human TNFα antibody, or antigen-binding portion thereof, and an anti-depressant agent. More in particular, in one embodiment, the invention provides an isolated human anti-TNFα antibody, or an antigen binding portion thereof, for use in the treatment of depression, wherein the treatment of depression further comprises administering an antidepressant agent, wherein the human anti-TNFα antibody, or the antigen binding portion thereof, is subcutaneously administered to the subject and wherein the subcutaneous administration excludes perispinal administration. In another embodiment, the combination therapy of the invention comprises administration of D2E7 and an antidepressant:

The term "concomitant" as in the phrase "concomitant therapeutic treatment" includes administering an agent in the presence of a second agent. A concomitant therapeutic treatment method includes methods in which the first, second, third, or additional agents are co-administered. A concomitant therapeutic treatment method also includes methods in which the first or additional agents are administered in the presence of a second or additional agents, wherein the second or additional agents, for example, may have been previously administered. A concomitant therapeutic treatment method may be executed step-wise by different actors. For example, one actor may administer to a subject a first agent and a second actor may administer to the subject a second agent, and the administering steps may be executed at the same time, or nearly the same time, or at distant times, so long as the first agent (and additional agents) are after administration in the presence of the second agent (and additional agents). The actor and the subject maybe the same entity (*e.g*., human).

The term "combination therapy", as used herein, refers to the administration of two or more therapeutic substances, i.e. the anti-TNFα antibody and another drug. The other drug(s) may be administered concomitant with, prior to, or following the administration of a human TNFα antibody, or antigen-binding portion thereof.

As used herein, the term "depression" refers to a clinical syndrome that includes a persistent sad mood or loss of interest in activities. The Diagnostic and Statistical Manual of Mental Disorders (DSM-IV-TR) criteria can be used to diagnose patients as suffering from depression (American Psychiatric Association. Diagnostic and Statistical Manual of Mental Disorders-Text Revision. 4th ed. Washington: American Psychiatric Association; 2000).

More in particular, in one embodiment, the invention provides an isolated human anti-TNFα antibody, or an antigen binding portion thereof, for use in the treatment of depression, wherein the treatment of depression further comprises administering an antidepressant agent., wherein the human anti-TNFα antibody, or the antigen binding portion thereof, is subcutaneously administered to the subject and wherein the subcutaneous administration excludes perispinal administration.

Similarly, the International Classification of Disease, version 10 (IDC-10), of the World Health Organization, lists criteria for depression. Examples of types of depression or depressive disorders include, but are not limited to, dysthmic disorder, bipolar disorder, major depression, and cyclothymic disorder. The term "TNFα-mediated depression" or "TNFα-related depression" refers to depression which is associated with increased TNFα activity or levels. In one embodiment, TNFα-mediated depression is identified in a subject who has an increase in TNFα serum levels relative to levels normally seen in non-depressed subjects. In another embodiment, a subject having a disorder associated with detrimental TNFα activity, such as, but not limited to, rheumatoid arthritis, Crohn's disease, and psoriasis, may also have TNFα-mediated depression. In one embodiment, the instant invention provides an isolated human anti-TNFα antibody or an antigen binding portion thereof, for use in the treatment of depression, wherein the human anti- TNFα antibody or the antigen binding portion thereof is subcutaneously administered to the subject and the subcutaneous administration excludes perispinal administration. In another embodiment, the antibody for use in the invention is used to treat depression in a subject having an additional disorder in which TNFα activity is detrimental.

The term "systemic administration" as used herein, refers to a method of administering a TNFα inhibitor, such as a TNFα antibody, or antigen-binding fragment thereof, to a subject via the blood stream. Systemic administration provides inhibition of peripheral TNFα in contrast to direct administration to the central nervous system which provides for inhibition of central TNFα. The term "systemic administration" excludes perispinal administration of the TNFα inhibitor, e.g., a TNFα antibody, for methods of treatment of depression. An example of systemic administration includes subcutaneous administration.

The term "kit" as used herein refers to a packaged product comprising components with which to administer a TNFα inhibitor, such as a TNFα antibody for treatment of a TNFα-mediated depression. The kit preferably comprises a box or container that holds the components of the kit. The box or container is affixed with a label or a Food and Drug Administration approved protocol for treating depression. The box or container holds components of the invention which are preferably contained within plastic, polyethylene, polypropylene, ethylene, or propylene vessels. The vessels can be capped-tubes or bottles. The kit can also include instructions for administering the TNFα antibody of the invention. In one embodiment the kit of the invention includes the formulation comprising the human antibody D2E7, as described in PCT/IB03/04502 and U.S. Appln. No. 10/222140.

Various aspects of the invention are described in further detail herein.

### II. TNFα Inhibitors of the Invention

This invention provides an isolated human anti-TNFα antibody, or an antigen binding portion thereof, for use in the treatment of depression, wherein the human anti-TNFα antibody, or the antigen binding portion thereof, is subcutaneously administered to the subject and wherein the subcutaneous administration excludes perispinal administration.

In one embodiment, the treatment includes administration of isolated human antibodies, or antigen-binding portions thereof, that bind to human TNFα with high affinity and a low off rate, and have a high neutralizing capacity. Preferably, the human antibodies of the invention are recombinant, neutralizing human anti-hTNFα antibodies. The most preferred recombinant, neutralizing antibody of the invention is referred to herein as D2E7, also referred to as HUMIRA^{®} and adalimumab (the amino acid sequence of the D2E7 VL region is shown in SEQ ID NO: 1; the amino acid sequence of the D2E7 VH region is shown in SEQ ID NO: 2). The properties of D2E7 (adalimumab / HUMIRA^{®}) have been described in Salfeld et al., U.S. Patent Nos. 6,090,382, 6,258,562, and 6,509,015. The invention may also be performed using chimeric and humanized murine anti-hTNFα antibodies which have undergone clinical testing for treatment of rheumatoid arthritis; (see *e.g.,* Elliott, M.J., et al. (1994) Lancet 344:1125-1127; Elliot, MJ., et al. (1994) Lancet 344:1105-1110; Rankin, E.C., et al. (1995) Br. J. Rheumatol. 34:334-342).

In one embodiment, the method of treating depression used in the invention includes the subcutaneous administration which excludes perispinal administration of D2E7 antibodies and antibody portions, D2E7-related antibodies and antibody portions, and other human antibodies and antibody portions with equivalent properties to D2E7, such as high affinity binding to hTNFα with low dissociation kinetics and high neutralizing capacity. In one embodiment, the invention provides treatment with an isolated human antibody, or an antigen-binding portion thereof, that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. More preferably, the isolated human antibody, or antigen-binding portion thereof, dissociates from human TNFα with a K_{off} of 5 x 10⁻⁴ s⁻¹ or less, or even more preferably, with a K_{off} of 1 x 10⁻⁴ s⁻¹ or less. More preferably, the isolated human antibody, or antigen-binding portion thereof, neutralizes human TNFα cytotoxicity in a standard *in vitro* L929 assay with an IC₅₀ of 1 x 10⁻⁸ M or less, even more preferably with an IC₅₀ or x 10⁻⁹ M or less and still more preferably with an IC₅₀ of 1 x 10⁻¹⁰ M or less. In a preferred embodiment, the antibody is an isolated human recombinant antibody, or an antigen-binding portion thereof.

It is well known in the art that antibody heavy and light chain CDR3 domains play an important role in the binding specificity/affinity of an antibody for an antigen. Accordingly, in another aspect, the invention pertains to methods of treating depression by administering human antibodies that have slow dissociation kinetics for association with hTNFα and that have light and heavy chain CDR3 domains that structurally are identical to or related to those of D2E7. Position 9 of the D2E7 VL CDR3 can be occupied by Ala or Thr without substantially affecting the K_{off}. Accordingly, a consensus motif for the D2E7 VL CDR3 comprises the amino acid sequence: Q-R-Y-N-R-A-P-Y-(T/A) (SEQ ID NO: 3). Additionally, position 12 of the D2E7 VH CDR3 can be occupied by Tyr or Asn, without substantially affecting the K_{off}. Accordingly, a consensus motif for the D2E7 VH CDR3 comprises the amino acid sequence: V-S-Y-L-S-T-A-S-S-L-D-(Y/N) (SEQ ID NO: 4). Moreover, as demonstrated in Example 2 of U.S. Patent No. 6,090,382, the CDR3 domain of the D2E7 heavy and light chains is amenable to substitution with a single alanine residue (at position 1,4,5,7 or 8 within the VL CDR3 or at position 2, 3, 4, 5, 6, 8, 9, 10 or 11 within the VH CDR3) without substantially affecting the K_{off}. Still further, the skilled artisan will appreciate that, given the amenability of the D2E7 VL and VH CDR3 domains to substitutions by alanine, substitution of other amino acids within the CDR3 domains may be possible while still retaining the low off rate constant of the antibody, in particular substitutions with conservative amino acids. Preferably, no more than one to five conservative amino acid substitutions are made within the D2E7 VL and/or VH CDR3 domains. More preferably, no more than one to three conservative amino acid substitutions are made within the D2E7 VL and/or VH CDR3 domains. Additionally, conservative amino acid substitutions should not be made at amino acid positions critical for binding to hTNFα. Positions 2 and 5 of the D2E7 VL CDR3 and positions 1 and 7 of the D2E7 VH CDR3 appear to be critical for interaction with hTNFα and thus, conservative amino acid substitutions preferably are not made at these positions (although an alanine substitution at position 5 of the D2E7 VL CDR3 is acceptable, as described above) (*see* U.S. Patent No. 6,090,382).

Accordingly, in another embodiment, the invention for use in methods of treating depression by subcutaneous administration which excludes perispinal administration of an isolated human antibody, or antigen-binding portion thereof. The antibody or antigen-binding portion thereof preferably contains the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

More preferably, the antibody, or antigen-binding portion thereof, dissociates from human TNFα with a K_{off} of 5 x 10⁻⁴ s⁻¹ or less. Even more preferably, the antibody, or antigen-binding portion thereof, dissociates from human TNFα with a K_{off} of 1 x 10⁻⁴ s⁻¹ or less.

In yet another embodiment, the invention is for use in methods of treating depression by subcutaneous administration which excludes perispinal administration of an isolated human antibody, or antigen-binding portion thereof. The antibody or antigen-binding portion thereof preferably contains a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and with a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. Preferably, the LCVR further has a CDR2 domain comprising the amino acid sequence of SEQ ID NO: 5 (*i.e.*, the D2E7 VL CDR2) and the HCVR further has a CDR2 domain comprising the amino acid sequence of SEQ ID NO: 6 (*i.e.*, the D2E7 VH CDR2). Even more preferably, the LCVR further has CDR1 domain comprising the amino acid sequence of SEQ ID NO: 7 (*i.e.,* the D2E7 VL CDR1) and the HCVR has a CDR1 domain comprising the amino acid sequence of SEQ ID NO: 8 (*i.e.*, the D2E7 VH CDR1). The framework regions fpr VL preferably are from the V_{κ}I human germline family, more preferably from the A20 human germline Vk gene and most preferably from the D2E7 VL framework sequences shown in Figures 1A and 1B of U.S. Patent No. 6,090,382. The framework regions for VH preferably are from the V_{H}3 human germline family, more preferably from the DP-31 human germline VH gene and most preferably from the D2E7 VH framework sequences shown in Figures 2A and 2B of U.S. Patent No. 6,090,382.

Accordingly, in another embodiment, the invention is for use in methods of treating depression by the subcutaneous administration which excludes perispinal administration of an isolated human antibody, or antigen-binding portion thereof. The antibody or antigen-binding portion thereof preferably contains a light chain variable region (LCVR) comprising the amino acid sequence of SEQ ID NO: 1 (*i.e.*, the D2E7 VL) and a heavy chain variable region (HCVR) comprising the amino acid sequence of SEQ ID NO: 2 (*i.e.*, the D2E7 VH). In certain embodiments, the antibody comprises a heavy chain constant region, such as an IgG1, IgG2, IgG3, IgG4, IgA, IgE, IgM or IgD constant region. Preferably, the heavy chain constant region is an IgG1 heavy chain constant region or an IgG4 heavy chain constant region. Furthermore, the antibody can comprise a light chain constant region, either a kappa light chain constant region or a lambda light chain constant region. Preferably, the antibody comprises a kappa light chain constant region. Alternatively, the antibody portion can be, for example, a Fab fragment or a single chain Fv fragment.

In still other embodiments, the methods of treating depression used in the invention comprise subcutaneous administration which excludes perispinal administration of an isolated human antibody, or an antigen-binding portions thereof, containing D2E7-related VL and VH CDR3 domains. For example, antibodies, or antigen-binding portions thereof, with a light chain variable region (LCVR) having a CDR3 domain comprising an amino acid sequence selected from the group consisting of SEQ ID NO: 3, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26 or with a heavy chain variable region (HCVR) having a CDR3 domain comprising an amino acid sequence selected from the group consisting of SEQ ID NO: 4, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34 and SEQ ID NO: 35.

In another embodiment, the method for use in the invention include treating depression by subcutaneous administration which excludes perispinal administration of a TNFα inhibitor, including, but not limited to, an anti-TNFα antibody, or a fragment thereof, including infliximab (Remicade^{®}, Johnson and Johnson; described in U.S. Patent No. 5,656,272, incorporated by reference herein), CDP571 (a humanized monoclonal anti-TNF-alpha IgG4 antibody), CDP 870 (a humanized monoclonal anti-TNF-alpha antibody fragment), an anti-TNF dAb (Peptech), CNTO 148 (golimumab; Medarex and Centocor, see WO 02/12502), and adalimumab (Humira^{®} Abbott Laboratories, a human anti-TNF mAb, described in US 6,090,382 as D2E7). Other examples include etanercept (described in WO 91/03553 and WO 09/406476), soluble TNF receptor Type I, a pegylated soluble TNF receptor Type I (PEGs TNF-R1), or p55TNFR1gG (Lenercept). In another embodiment, the TNFα inhibitor is a recombinant TNF binding protein (r-TBP-I) (Serono).

The TNFα antibody for use in the invention may be modified for improved treatment of depression. In some embodiments, the TNFα antibody or antigen binding fragments thereof, is chemically modified to provide a desired effect. For example, pegylation of antibodies and antibody fragments of the invention may be carried out by any of the pegylation reactions known in the art, as described, for example, in the following references: Focus on Growth Factors 3:4-10 (1992); EP 0154 316; and EP 0 401384.

Preferably, the pegylation is carried out via an acylation reaction or an alkylation reaction with a reactive polyethylene glycol molecule (or an analogous reactive water-soluble polymer). A preferred water-soluble polymer for pegylation of the antibodies and antibody fragments of the invention is polyethylene glycol (PEG). As used herein, "polyethylene glycol" is meant to encompass any of the forms of PEG that have been used to derivatize other proteins, such as mono (CI-CIO) alkoxy- or aryloxy-polyethylene glycol.

Methods for preparing pegylated antibodies and antibody fragments for use in the invention will generally comprise the steps of (a) reacting the antibody or antibody fragment with polyethylene glycol, such as a reactive ester or aldehyde derivative of PEG, under conditions whereby the antibody or antibody fragment becomes attached to one or more PEG groups, and (b) obtaining the reaction products. It will be apparent to one of ordinary skill in the art to select the optimal reaction conditions or the acylation reactions based on known parameters and the desired result.

Pegylated antibodies and antibody fragments may generally be used to treat TNFα-related disorders as encompassed by the invention by subcutaneous administration which excludes perispinal administration of the TNFα antibodies and antibody fragments described herein. Generally the pegylated antibodies and antibody fragments have increased half-life, as compared to the nonpegylated antibodies and antibody fragments. The pegylated antibodies and antibody fragments may be employed alone, together, or in combination with other pharmaceutical compositions.

In yet another embodiment of the invention; TNFα antibodies or fragments thereof can be altered wherein the constant region of the antibody is modified to reduce at least one constant region-mediated biological effector function relative to an unmodified antibody. To modify an antibody of the invention such that it exhibits reduced binding to the Fc receptor, the immunoglobulin constant region segment of the antibody can be mutated at particular regions necessary for Fc receptor (FcR) interactions (see *e.g.,* Canfield, S.M. and S.L. Morrison (1991) J. Exp. Med. 173:1483-1491; and Lund, J. et al. (1991) J. of Immunol. 147:2657-2662). Reduction in FcR binding ability of the antibody may also reduce other effector functions which rely on FcR interactions, such as opsonization and phagocytosis and antigen-dependent cellular cytotoxicity.

An antibody or antibody portion for use in the methods of the invention can be derivatized or linked to another functional molecule (*e*.*g*., another peptide or protein). Accordingly, the antibodies and antibody portions for use in the invention are intended to include derivatized and otherwise modified forms of the human anti-hTNFα antibodies described herein, including immunoadhesion molecules. For example, an antibody or antibody portion for use in the invention can be functionally linked (by chemical coupling, genetic fusion, noncovalent association or otherwise) to one or more other molecular entities, such as another antibody (*e*.*g*., a bispecific antibody or a diabody), a detectable agent, a cytotoxic agent, a pharmaceutical agent, and/or a protein or peptide that can mediate associate of the antibody or antibody portion with another molecule (such as a streptavidin core region or a polyhistidine tag).

One type of derivatized antibody is produced by crosslinking two or more antibodies (of the same type or of different types, *e.g.,* to create bispecific antibodies). Suitable crosslinkers include those that are heterobifunctional, having two distinctly reactive groups separated by an appropriate spacer (*e.g.,* m-maleimidobenzoyl-N-hydroxysuccinimide ester) or homobifunctional (*e*.*g*., disuccinimidyl suberate). Such linkers are available from Pierce Chemical Company, Rockford, IL.

Useful detectable agents with which an antibody or antibody portion for use in the invention may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a colored reaction product, which is detectable. An antibody may also be derivatized with biotin, and detected through indirect measurement of avidin or streptavidin binding.

An antibody, or antibody portion, for use in the invention can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel, F.M. et al. (eds.) Current Protocols in Molecular Biology, Greene Publishing Associates, (1989) and in U.S. Patent No. 4,816,397 by Boss et al.

To express D2E7 or a D2E7-related antibody, DNA fragments encoding the light and heavy chain variable regions are first obtained. These DNAs can be obtained by amplification and modification of germline light and heavy chain variable sequences using the polymerase chain reaction (PCR). Germline DNA sequences for human heavy and light chain variable region genes are known in the art (see *e.g.,* the "Vbase" human germline sequence database; see also Kabat, E.A., et al. (1991) Sequences of Proteins of Immunological Interest, Fifth Edition, U.S. Department of Health and Human Services, NIH Publication No. 91-3242; Tomlinson, I.M., et al. (1992) "The Repertoire of Human Germline VH Sequences Reveals about Fifty Groups of VH Segments with Different Hypervariable Loops" J. Mol. Biol. 227:776-798; and Cox, I.P.L. et al. (1994) "A Directory of Human Germ-line V78 Segments Reveals a Strong Bias in their Usage" Eur. J. Immunol. 24:827-836;

To obtain a DNA fragment encoding the heavy chain variable region of D2E7, or a D2E7-related antibody, a member of the V_{H}3 family of human germline VH genes is amplified by standard PCR. Most preferably, the DP-31 VH germline sequence is amplified. To obtain a DNA fragment encoding the light chain variable region of D2E7, or a D2E7-related antibody, a member of the V_{K}I family of human germline VL genes is amplified by standard PCR. Most preferably, the A20 VL germline sequence is amplified. PCR primers suitable for use in amplifying the DP-31 germline VH and A20 germline VL sequences can be designed based on the nucleotide sequences disclosed in the references cited *supra,* using standard methods.

Once the germline VH and VL fragments are obtained, these sequences can be mutated to encode the D2E7 or D2E7-related amino acid sequences disclosed herein. The amino acid sequences encoded by the germline VH and VL DNA sequences are first compared to the D2E7 or D2E7-related VH and VL amino acid sequences to identify amino acid residues in the D2E7 or D2E7-related sequence that differ from germline. Then, the appropriate nucleotides of the germline DNA sequences are mutated such that the mutated germline sequence encodes the D2E7 or D2E7-related amino acid sequence, using the genetic code to determine which nucleotide changes should be made. Mutagenesis of the germline sequences is carried out by standard methods, such as PCR-mediated mutagenesis (in which the mutated nucleotides are incorporated into the PCR primers such that the PCR product contains the mutations) or site-directed mutagenesis.

Once DNA fragments encoding D2E7 or D2E7-related VH and VL segments are obtained (by amplification and mutagenesis of germline VH and VL genes, as described above), these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see *e*.*g*., Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see *e.g.,* Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, *e*.*g*., encoding the amino acid sequence (Gly₄-Ser)₃, such that the VH and VL sequences can be expressed as a contiguous single-chain protein, with the VL and VH regions joined by the flexible linker (see *e.g*., Bird et al. (1988) Science 242:423-426; Huston et al. (1988) Proc. Natl. Acad. Sci. USA 85:5879-5883; McCafferty et al., Nature (1990) 348:552-554).

To express the antibodies, or antibody portions of the invention, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (*e*.*g*., ligation of complementary restriction sites on the antibody gene fragment and vector, or blunt end ligation if no restriction sites are present). Prior to insertion of the D2E7 or D2E7-related light or heavy chain sequences, the expression vector may already carry antibody constant region sequences. For example, one approach to converting the D2E7 or D2E7-related VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e*., a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements (*e.g*., polyadenylation signals) that control the transcription or translation of the antibody chain genes. Such regulatory sequences are described, for example, in Goeddel; Gene Expression Technology: Methods in Enzymology 185, Academic Press, San Diego, CA (1990). It will be appreciated by those skilled in the art that the design of the expression vector, including the selection of regulatory sequences may depend on such factors as the choice of the host cell to be transformed, the level of expression of protein desired, *etc.* Preferred regulatory sequences for mammalian host cell expression include viral elements that direct high levels of protein expression in mammalian cells, such as promoters and/or enhancers derived from cytomegalovirus (CMV (such as the CMV promoter/enhancer), Simian Virus 40 (SV40) (such as the SV40 promoter/enhancer), adenovirus, (*e*.*g*., the adenovirus major late promoter (AdMLP)) and polyoma. For further description of viral regulatory elements, and sequences thereof, see *e.g.,* U.S. Patent No. 5,168,062 by Stinski, U.S. Patent No. 4,510,245 by Bell et al*.* and U.S. Patent No. 4,968,615 by Schaffner et al.

In addition to the antibody chain genes and regulatory sequences, the recombinant expression vectors of the invention may carry additional sequences, such as sequences that regulate replication of the vector in host cells (*e*.*g*., origins of replication) and selectable marker genes. The selectable marker gene facilitates selection of host cells into which the vector has been introduced (see *e.g.,* U.S. Patents Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr⁻ host cells with methotrexate selection/amplification) and the *neo* gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e*.*g*., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M.A. and Wood, C. R. (1985) Immunology Today 6:12-13).

Preferred mammalian host cells for expressing the recombinant antibodies of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, *e*.*g*., as described in R.J. Kaufman and P.A. Sharp (1982) Mol. Biol. 159:601-621), NS0 myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments or scFv molecules. It is understood that variations on the above procedure are within the scope of the present invention. For example, it may be desirable to transfect a host cell with DNA encoding either the light chain or the heavy chain (but not both) of an antibody of this invention. Recombinant DNA technology may also be used to remove some or all of the DNA encoding either or both of the light and heavy chains that is not necessary for binding to hTNFα. The molecules expressed from such truncated DNA molecules are also encompassed by the antibodies of the invention. In addition, bifunctional antibodies may be produced in which one heavy and one light chain are an antibody of the invention and the other heavy and light chain are specific for an antigen other than hTNFα by crosslinking an antibody of the invention to a second antibody by standard chemical crosslinking methods.

In a preferred system for recombinant expression of an antibody, or antigen-binding portion thereof, for use in the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Recombinant human antibodies for use in the invention in addition to D2E7 or an antigen binding portion thereof, or D2E7-related antibodies disclosed herein can be isolated by screening of a recombinant combinatorial antibody library, preferably a scFv phage display library, prepared using human VL and VH cDNAs prepared from mRNA derived from human lymphocytes. Methodologies for preparing and screening such libraries are known in the art. In addition to commercially available kits for generating phage display libraries (*e.g*., the *Pharmacia Recombinant Phage Antibody System,* catalog no. 27-9400-01; and the Stratagene *Surf*ZAP^{™} phage display kit, catalog no. 240612), examples of methods and reagents particularly amenable for use in generating and screening antibody display libraries can be found in, for example, Ladner et al. U.S. Patent No. 5,223,409; Kang et al. PCT Publication No. WO 92/18619; Dower et al. PCT Publication No. WO 91/17271; Winter et al. PCT Publication No. WO 92/20791; Markland et al. PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) Bio/Technology 9:1370-1372; Hay et al. (1992) Hum Antibod Hybridomas 3:81-85; Huse et al. (1989) Science 246:1275-1281; McCafferty et al., Nature (1990) 348:552-554; Griffiths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrard et al. (1991) Bio/Technology 9:1373-1377; Hoogenboom et al. (1991) Nuc Acid Res 19:4133-4137; and Barbas et al. (1991) PNAS 88:7978-7982.

In a preferred embodiment, to isolate human antibodies with high affinity and a low off rate constant for hTNFα, a murine anti-hTNFα antibody having high affinity and a low off rate constant for hTNFα *(e.g.,* MAK 195, the hybridoma for which has deposit number ECACC 87 050801) is first used to select human heavy and light chain sequences having similar binding activity toward hTNFα, using the epitope imprinting methods described in Hoogenboom et al., PCT Publication No. WO 93/06213. The antibody libraries used in this method are preferably scFv libraries prepared and screened as described in McCafferty et al., PCT Publication No. WO 92/01047, McCafferty et al., Nature (1990) 348:552-554; and Griffiths et al., (1993) EMBO J 12:725-734. The scFv antibody libraries preferably are screened using recombinant human TNFα as the antigen.

Once initial human VL and VH segments are selected, "mix and match" experiments, in which different pairs of the initially selected VL and VH segments are screened for hTNFα binding, are performed to select preferred VL/VH pair combinations. Additionally, to further improve the affinity and/or lower the off rate constant for hTNFα binding, the VL and VH segments of the preferred VL/VH pair(s) can be randomly mutated, preferably within the CDR3 region of VH and/or VL, in a process analogous to the *in vivo* somatic mutation process responsible for affinity maturation of antibodies during a natural immune response. This *in vitro* affinity maturation can be accomplished by amplifying VH and VL regions using PCR primers complimentary to the VH CDR3 or VL CDR3, respectively, which primers have been "spiked" with a random mixture of the four nucleotide bases at certain positions such that the resultant PCR products encode VH and VL segments into which random mutations have been introduced into the VH and/or VL CDR3 regions. These randomly mutated VH and VL segments can be rescreened for binding to hTNFα and sequences that exhibit high affinity and a low off rate for hTNFα binding can be selected.

Following screening and isolation of an anti-hTNFα antibody for use in the invention from a recombinant immunoglobulin display library, nucleic acid encoding the selected antibody can be recovered from the display package (*e*.*g*., from the phage genome) and subcloned into other expression vectors by standard recombinant DNA techniques. If desired, the nucleic acid can be further manipulated to create other antibody forms of the invention (*e*.*g*., linked to nucleic acid encoding additional immunoglobulin domains, such as additional constant regions). To express a recombinant human antibody isolated by screening of a combinatorial library, the DNA encoding the antibody is cloned into a recombinant expression vector and introduced into a mammalian host cells, as described in further detail in above.

Methods of isolating human antibodies with high affinity and a low off rate constant for hTNFα are also described in U.S. Patent Nos. 6,090,382, 6,258,562, and 6,509,015.

### III. Use of the TNFα, Inhibitors for Treatment of Depression.

The invention provides methods of treating depression comprising inhibiting peripheral TNFα. The invention also provides an isolated human anti-TNFα antibody, or an antigen binding portion thereof, for use in the treatment of a subject suffering from or at risk of suffering from depression associated with TNF[alpha], wherein the human anti-TNFα antibody, or the antigen binding portion thereof, is subcutaneously administered to the subject and wherein the subcutaneous administration excludes perispinal administration. In one embodiment, the TNFα antibody is administered in combination with an additional therapeutic agent, such as an antidepressant agent. In one embodiment, the TNFα antibody is D2E7, also referred to as HUMIRA^{®} (adalimumab).

TNFα has been shown to be associated with depression. Depressed patients that responded to antidepressant treatment showed normalized values of TNFα, while non-responders showed elevated levels of TNFα, indicative of persistence of immune activation (Lanquillon et al., Neuropsychopharmacology (2000) 22:370-379 and Tuglu et al. (2003), Psychopharmacology 170:429-433). For example, elevated serum levels of TNFα have been observed in major depressed patients, and high levels of inflammatory markers are associated with increased risk of depressed mood (see Mikova et al.(2001), European Neuropsychopharmacology 11: 203-208 and Penninx et al. (2003), Biol Psychiatry 54:566-572). Thus, TNFα,-mediated depression is intended to include depressive disorders in which the presence of TNFα in a subject suffering from the depression has been shown to be or is suspected of being either responsible for the pathophysiology of the disorder or a factor that contributes to a worsening of the disorder. Accordingly, TNFα-mediated depression is a depression in which inhibition of TNFα activity is expected to alleviate the symptoms and/or progression of the depression, *e*.*g*., improve the overall mood of the affected individual, improve self-esteem of the subject. Such disorders may be evidenced, for example, by an increase in the concentration of TNFα in a biological fluid of a subject suffering from the disorder (*e*.*g*., an increase in the concentration of TNFα in serum, plasma, synovial fluid, *etc.* of the subject), which can be detected, for example, using an anti-TNFα antibody as deseribed above.

The term depression contemplates all diseases and conditions which are associated with depression including those classified in the IDC-10 and DSM-IV rating scales. Symptoms of depression include, but are not limited to, feeling sad, hopeless, worthless, or pessimistic. The Diagnostic and Statistical Manual of Mental Disorders

(DSM-IV-TR) criteria can be used to diagnose patients as suffering from depression (American Psychiatric Association. Diagnostic and Statistical Manual of Mental Disorders-Text Revision. 4th ed. Washington: American Psychiatric Association; 2000), incorporated by reference herein. Similarly, the International Classification of Disease, version 10 (IDC-10) (incorporated by reference herein), of the World Health Organzation, lists criteria for depression. Examples of types of depression or depressive disorders which may be treated by the methods of the invention include, but are not limited to, major depression, dysthymic disorder, cyclothymic disorder, bipolar disorder, and depressive episodes associated with other mood disorders, including seasonal mood disorders such as seasonal affective disorder, subsyndromal depression, single episode depression, post partum depression, and mood disorders due to a general medical condition, substance induced mood disorder, recurrent or treatment-resistant depression, child abuse induced depression, atypical depression, cyclothymia, menstrual-related dysphoria, depression associated with somatoform disorder, and treatment-resistant depression.

### A. Major Depression

In one embodiment, an isolated human anti-TNFα antibody, or an antigen binding portion thereof, for use in the treatment of major depression, wherein the.human anti-TNFα antibody, or the antigen binding portion thereof, is subcutaneously administered to the subject and wherein the subcutaneous administration excludes perispinal administration.

Major depression is also commonly referred to as unipolar depression and major depressive disorder. Major depression is characterized as a subject having five or more symptoms of depression for a specific time period, typically at least 2 weeks. In addition, people with major depression often have behavior changes, such as new eating and sleeping patterns, and may have thoughts of suicide. Various forms of major depression, including a single episode or recurrent major depression, may be treated using a human TNFα antibody, or antigen-binding fragment thereof. Refractory (or treatment resistant) major depression may also be treated with the methods of the invention. Treatment-resistant depression (TRD) is a common clinical occurrence among patients treated for major depressive disorder. Methods of identifying TRD are described in Souery et al. (2006) J Clin Psychiatry 67:16-22, incorporated by reference herein.

### B. Dysthmic Disorder

In one embodiment, the invention provides an isolated human anti-TNFα, antibody, or an antigen binding portion thereof, for use in the treatment of a dysthmic disorder, wherein the human anti-TNFα antibody, or the antigen binding portion thereof, is subcutaneously administered to the subject and wherein the subcutaneous administration excludes perispinal administration.

Dysthmic disorder, or dysthmia, is also commonly referred to as neurotic depression or chronic depression. Symptoms of dysthmia include, but are not limited to, poor appetite or overeating, insomnia or hypersomnia, low energy or fatigue, low self-esteem, poor concentration, and feelings of hopelessness. Symptoms of dysthmia are often not as severe in affected subjects as in other forms of depression.

Major depressive disorder and dysthymic disorder are differentiated based on chronicity, severity and persistence. In major depression the depressed mood is usually present for about two weeks. In dysthymic disorder the depressed mood is usually present most days over a period of about two years. Usually major depressive disorder is characterized by its sharp contrast to usual functioning. A person with a major depressive episode can be functioning and feeling normally and suddenly develops severe symptoms of depression. By contrast a person with dysthymic disorder has chronic depression with less severe symptoms than major depression for generally a longer time span.

### C. Cyclothymic Disorder

The invention also provides an isolated human anti-TNFα antibody, or an antigen binding portion thereof, for use in the treatment of a cyclothymic disorder, wherein the human anti-TNFα antibody, or the antigen binding portion thereof, is subcutaneously administered to the subject and wherein the subcutaneous administration excludes perispinal administration. Cyclothymic disorder, also called cyclothymia, is a mild form of bipolar disorder, characterized by alternating episodes of mood swings from mild or moderate depression to hypomania. Hypomania is defined as periods of elevated mood, euphoria, and excitement that do not cause the person to become disconnected from reality.

### D. Bipolar Disorders

A TNFα antibody, such as a human TNFα antibody, or antigen-binding fragment thereof, may also be used according to the invention to treat a subject having bipolar disorder, also referred to as manic depression and bipolar affective disorder. Bipolar disorder is characterized by periods of excitability (mania) alterriating with periods of depression. The "mood swings" between mania and depression can be very abrupt and may be intermittent.

Bipolar disorders can be categorized as either bipolar I disorder or bipolar II disorder. Bipolar I disorder is characterized by one or more manic episodes or mixed episodes and often one or more major depressive episodes. A depressive episode may last for several weeks or months, alternating with intense symptoms of mania that may last just as long. Between episodes, there may be periods of normal functioning. Symptoms may also be related to seasonal changes. Bipolar II disorder is characterized by one or more major depressive episodes accompanied by at least one hypomanic episode. Hypomanic episodes have symptoms similar to manic episodes, but are less severe. Between episodes, an affected subject may have periods of normal functioning. Symptoms of bipolar II disorder may also be related to seasonal changes. Issues and treatments involving bipolar depression are described in Thase (2005) Harv Rev Psychiatry. 2005 Sep-Oct;13(5):257-71.

All of the depressive disorders referred to above may be associated with additional features, including catatonic features, melancholic features, atypical features, and postpartum onset of the disorder.

TNFα antibodies may also be used to treat refractory or treatment resistant depression. Refractory depression may occur in patients who have been treated for depression, but have failed to respond to therapy.

Treatment of depression may also be determined using measures of serum levels of TNFα and/or depression symptoms (e.g. HAM-D) in depressed patients before and after completion of the treatment with Humira (adalimumab). Depression may be diagnosed by one of ordinary skill in the art through the use of an accepted index or scale which determines the depression status of an individual. In addition, any of the following indices / scales, as well as others known in the art, may be used to determine the efficacy of a TNFα inhibitor, e.g., a TNFα antibody, for treating depression in a subject in accordance with the invention. An improvement in the index or scale known in the art, such as those described below, indicates that the TNFα inhibitor is effective for the treatment of depression. Such improvements may be determined by comparing the pretreatment and post-treatment score of the subject having depression.

An example of such an index is the Hamilton depression rating scale (HAM-D) (Journal of Neurology Neurosurgery and Psychiatry 23:56-62,1960), incorporated by reference herein. The 24-item HAM-D questionnaire is used to rate the severity of a patient's depression and evaluate the efficacy of antidepressant therapy. HAM-D scores are generally interpreted as follows: very severe, >23; severe, 19-22; moderate, 14-18; mild, 8-13; and no depression, 0-7. Response to treatment can be defined as patients with a 50% or more decrease in HAM-D score. Remission is defined as a HAM-D score of 7 or less. In one embodiment, a TNFα inhibitor, e.g., a TNFα antibody, is administered systemically for the treatment of depression, wherein treatment of depression is determined by an improvement in the HAM-D score of the subject, e.g., a HAM-D score of ≤ 7 following treatment.

Another example of an efficacy variable which may be used in the method of the invention, includes the Hamilton anxiety scale (HAM-A) (British Journal of Medical Psychology, British Journal of Medical Psychology 32:50-55,1959), incorporated by reference herein. The HAM-A scale consists of 14 items, each defined by series of symptoms, scored on a scale of 0 (not present) to 4. HAM-A scores are generally interpreted as follows: mild, ≤17; mild to moderate, 18-24; and moderate to severe, 25-30.

The Clinical Global Impression (CGI) (Guy W (1976). Clinical Global Impressions ECDEU Assessment Manual for Psychopharmacology, Revised (DHEW Publ. No. ADM 76-338). National Institute of Mental Health: Rockville, MD. Pp.218-222), incorporated by reference herein, may also be used in the methods of the invention to determine treatment of depression by subcutaneously administering a TNFα inhibitor, e.g., a TNFα antibody, wherein the subcutaneous administration excludes perispinal administration. The CGI scale refers to the global severity of illness and change. in the clinical condition over time. The CGI scale consists of three global subscales: severity of illness, global improvement and efficacy index. Severity of illness and global improvement are measured on a 7-point scale from 1 ('normal', not ill) to 7 (extremely ill), while the efficacy index involves a rating of the interaction of therapeutic effectiveness and side effects. Calculation of the efficacy index requires division of the therapeutic effect score by the side effect score. Improvement is generally defined as the clinical distance between the individual's current condition and that prior to the start of treatment, with the scores as follows: CGI-Global Improvement score of 1 (very much improved), 2 (much improved), 3 (minimally improved) or 4 (no change).

Another efficacy scale which may be used in the method used in the invention, is the Patient Global Impression of Improvement (PGI) which measures the degree of improvement since randomization at the time of the assessment The scores are interpreted as follows with respect to the subject's depressed state: 1, very much better; 2, much better; 3, a little better; 4, about the same; 5, a little worse; 6, much worse; and 7, very much worse.

Further examples of indices used to determine the depression status of an individual include the Bech-Rafaelsen Melancholic Scale (MES) (Acta Psychiatrica Scandinavica 106:252-64, 2002), the Montgomery-Asberg depression rating scale (MADRS) (British Journal of Psychiatry 134:382-389,1979), the major depression index (MDI) (Journal of Affective Disorders 66:159-164, 2001), the Beck depression index (BDI) (Archives of General Psychiatry 4:561-571,1961), and the hospital anxiety depression scale (HAD) (Acta Psychiatrica Scandinavica 67:361-370,1983).

Quality of life questionnaires may also used to determine efficacy. In general, quality of life questionnaires measure social functioning. Examples of quality of life questionnaires include: the Social and Occupational Functioning Scale (SOFAS), the Sheehan Disability Scale, the Social Adjustment Scale - Self Report (SAS-SR), the Social Adaptation Self-Evaluation Scale (SASS), the Short-Form Health Survey (SF-36), the Psychological General Well-Being Scale (PGWB) and the WHO-Five Well-Being Index (WHO-5).

### E. Depression and Other Disorders

The methods used in the invention may also be used to treat depression which is associated with another disorder, especially a disorder in which TNFα activity is detrimental. Other types of disorders in which TNFα activity is detrimental in which the affected subject may also suffer from depression include, rheumatoid arthritis, ankylosing spondylitis, Crohn's disease, psoriasis, and psoriatic arthritis. Other examples of disorders which may be associated with depression include coronary heart disease, a neurodegenerative disease, such as a stroke, an infectious disease, and an autoimmune disorder. Examples of autoimmune disorders which may be treated using the method of the invention include psoriasis, psoriatic arthritis, and rheumatoid arthritis. Examples of intestinal disorders which may be treated using the method of the invention include inflammatory bowel disease and Crohn's disease. Furthermore, the depressed subject may have Behcet's disease, asthma, and Niemann-Pick disease. TNF-α-related disorders as described in U.S. Appln No. 10/622932, WO 2004009776, PCT/US97/02219, and U.S. Pat. No. 6,258,562. may also be treated with the methods used in the invention.

### IV. Pharmaceutical Compositions and Pharmaceutical Administration

### A. Compositions

Antibodies and antibody-portions for use in the treatment and preventive methods of the invention, can be incorporated into pharmaceutical compositions suitable for subcutaneous administration which excludes perispinal administration to a subject with depression. Typically, the pharmaceutical composition comprises an antibody, antibody portion, and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, itis preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservative or buffers, which enhance the shen life or effectiveness of the antibody, or antibody portion.

The compositions for use in the methods of the invention may be in a variety of forms. These include, for example, liquid, semi-solid and solid dosage forms, such as liquid solutions (*e*.*g*., injectable and infusible solutions), dispersions or suspensions, or powders, liposomes. The preferred form depends on the intended mode of administration and therapeutic application. Typical preferred compositions are in the form of injectable or infusible solutions, such as compositions similar to those used for passive immunization of humans with other antibodies. The mode of administration is subcutaneous administration which excludes perispinal administration.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (*i*.*e*., antibody, antibody portion, or other TNFα inhibitor) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

Supplementary active compounds can also be incorporated into the compositions. In certain embodiments, an antibody or antibody portion for use in the methods of the invention is coformulated with and/or coadministered with one or more additional therapeutic agents, including an antidepressant agent. For example, an anti-hTNFα antibody or antibody portion of the invention may be coformulated and/or coadministered with one or more additional antibodies that bind other targets (*e*.*g*., antibodies that bind other cytokines or that bind cell surface molecules), one or more cytokines, soluble TNFα receptor (see *e*.*g*., PCT Publication No. WO 94/06476) and/or one or more chemical agents that inhibit hTNFα production or activity (such as cyclohexane-ylidene derivatives as described in PCT Publication No. WO 93/19751) or any combination thereof. Furthermore, one or more antibodies of the invention may be used in combination with two or more of the foregoing therapeutic agents. Such combination therapies may advantageously utilize lower dosages of the administered therapeutic agents, thus avoiding possible side effects, complications or low level of response by the patient associated with the various monotherapies.

In one embodiment, the invention includes pharmaceutical compositions comprising an effective amount of a TNFα inhibitor and a pharmaceutically acceptable carrier, wherein the effective amount of the TNFα inhibitor may be effective to treat depression. In one embodiment, the antibody or antibody portion for use in the methods of the invention is incorporated into a pharmaceutical formulation as described in PCT/IB03/04502 and U.S. Appln. No. 10/222140. This formulation includes a concentration 50 mg/ml of the antibody D2E7, wherein one prefilled syringe contains 40 mg of antibody for subcutaneous injection for treatment of depression. In another embodiment, the formulation of the invention includes D2E7 and an antidepressant.

The antibody D2E7 may also be administered in combination with an antidepressant agent for the treatment of depression. In one embodiment of the invention, D2E7 and an antidepressant agent are co-administered for treatment of depression. In another embodiment, D2E7 and an antidepressant agent are co-formulated for treatment of depression.

In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g*., Sustained and Controlled Release Drug Delivery Systems, J.R. Robinson, ed., Marcel Dekker, Inc., New York, 1978.

The TNFα antibodies of the invention can also be administered in the form of protein crystal formulations which include a combination of protein crystals encapsulated within a polymeric carrier to form coated particles. The coated particles of the protein crystal formulation may have a spherical morphology and be microspheres of up to 500 micro meters in diameter or they may have some other morphology and be microparticulates. The enhanced concentration of protein crystals allows the antibody of the invention to be delivered subcutaneously where subcutaneous administration excludes perispinal administration. In one embodiment, the TNFα antibodies of the invention are delivered via a protein delivery system, wherein one or more of a protein crystal formulation or composition, is administered to a subject with a TNFα-related disorder. Compositions and methods of preparing stabilized formulations of whole antibody crystals or antibody fragment crystals are also described in WO 02/072636.

In one embodiment, a formulation comprising the crystallized antibody fragments described in PCT/IB03/04502 and U.S. Appln. No. 10/222140, are used to treat a TNFα-related disorder using the multiple-variable dose methods of the invention.

### B. Administration

The invention provides an isolated human anti-TNFα antibody, or an antigen binding portion thereof, for use in the treatment of depression inhibiting peripheral TNF[alpha], wherein the human anti-TNFα antibody, or the antigen binding portion thereof, is subcutaneously administered to the subject and wherein the subcutaneous administration excludes perispinal administration.

Antibodies used to treat depression are administered to a subject having depression such that peripheral activity of TNFα is inhibited.

The TNFα antibody or antibody portion is administered via subcutaneous administration to the subject wherein the subcutaneous administration excludes perispinal administration. The location of the administration is preferably on the subject's extremities, i.e., the thighs.

Dosage regimens may be adjusted to provide the optimum desired response (*e*.*g*., a therapeutic or prophylactic response). For example, a single bolus may be administered, several divided doses may be administered over time or the dose may be proportionally reduced or increased as indicated by the exigencies of the therapeutic situation. It is especially advantageous to formulate subcutaneous compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

The pharmaceutical compositions for use in the invention may include a "therapeutically effective amount" or a "prophylactically effective amount" of an antibody or antibody portion of the invention. A "therapeutically effective amount" refers to an amount effective, at dosages and for periods of time necessary, to achieve the desired therapeutic result. A therapeutically effective amount of the antibody, antibody portion, or other TNFα inhibitor may vary according to factors such as the disease state, age, sex, and weight of the individual, and the ability of the antibody, antibody portion, other TNFα inhibitor to elicit a desired response in the individual. A therapeutically effective amount is also one in which any toxic or detrimental effects of the antibody, antibody portion, or other TNFα inhibitor are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

An exemplary, non-limiting range for a therapeutically or prophylactically effective amount of an antibody or antibody portion for use in the invention, such as the anti-TNFα antibody D2E7, is 10-180 mg, more preferably 20-160 mg and most preferably about 80 mg. In one embodiment, the therapeutically effective amount of an antibody or portion thereof for use in the invention is 40 mg. In another embodiment, the therapeutically effective amount of an antibody or portion thereof for use in the methods of the invention is 80 mg. In still another embodiment, the therapeutically effective amount of an antibody or portion thereof for use in the methods of the invention is 160 mg. Ranges intermediate to the above recited dosages, *e.g.* about 78.5 - 81.5, are also intended to be part of this invention. For example, ranges of values using a combination of any of the above recited values as upper and/or lower limits are intended to be included.

In another embodiment, the invention provides an isolated human anti-TNFα antibody, or an antigen binding portion thereof, for use in the treatment of depression, wherein the human anti-TNFα antibody, or the antigen binding portion thereof, is subcutaneously administered to the subject and wherein the subcutaneous administration excludes perispinal administration and wherein the treatment is a single dose method for treating depression, comprising administering to a subject in need thereof a single dose of a TNFα human antibody. In one embodiment, the anti-TNFα antibody D2E7. The single dose of anti-TNFα antibody can be any therapeutically or prophylactically effective amount. In one embodiment, a subject is administered either a 20 mg, a 40 mg, or an 80 mg single dose of D2E7.

Multiple variable dose methods of treatment or prevention can also be used, and are described in U.S. application no. 11/104117.

It is to be noted that dosage values may vary with the type and severity of the type of depression to be alleviated. It is to be further understood that for any particular subject, specific dosage regimens should be adjusted over time according to the individual need and the professional judgment of the person systemically administering or supervising the administration of the compositions, and that dosage ranges set forth herein are exemplary only and are not intended to limit the scope or practice of the invention.

### C. Kits

The instant disclosure also pertains to packaged pharmaceutical compositions or kits for administering anti-TNF antibodies for use in the methods of the invention. In one embodiment of the instant disclosures, the kit comprises an antibody and instructions for subcutaneous administration which excludes perispinal administration for treatment of depression, e.g., a TNFα antibody. The instructions may describe how, and when, *e.g*., at week 0 and week 2, the different doses of TNFα antibody and/or the additional therapeutic agent shall be administered to a subject for treatment.

Another aspect of the instant disclosure pertains to kits containing a pharmaceutical composition comprising an anti-TNFα antibody and a pharmaceutically acceptable carrier, and one or more pharmaceutical compositions each comprising a drug useful for treating depression and a pharmaceutically acceptable carrier. Alternatively, the kit comprises a single pharmaceutical composition comprising an anti-TNFα antibody, one or more drugs useful for treating depression and a pharmaceutically acceptable carrier. The kits contain instructions for dosing of the pharmaceutical compositions for the treatment of depression in which the subcutaneous administration which excludes perispinal administration of an anti-TNFα antibody is beneficial.

The package or kit alternatively can contain the TNFα antibody and it can be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

### D. Additional therapeutic agents

The instant disclosure pertains to pharmaceutical compositions and methods of use thereof for the treatment of depression. The pharmaceutical compositions comprise a first agent that prevents or treats depression. The pharmaceutical composition also may comprise a second agent that is an active pharmaceutical ingredient; that is, the second agent is therapeutic and its function is beyond that of an inactive ingredient, such as a pharmaceutical carrier, preservative, diluent, or buffer. The second agent may be useful in treating or preventing depression. The second agent may diminish or treat at least one symptom(s) associated with the depression. The first and second agents may exert their biological effects by similar or unrelated mechanisms of action; or either one or both of the first and second agents may exert their biological effects by a multiplicity of mechanisms of action. A pharmaceutical composition may also comprise a third compound, or even more yet, wherein the third (and fourth, etc.) compound has the same characteristics of a second agent.

It should be understood that the pharmaceutical compositions described herein may have the first and second, third, or additional agents in the same pharmaceutically acceptable carrier or in a different pharmaceutically acceptable carrier for each described embodiment. It further should be understood that the first, second, third and additional agent may be administered simultaneously or sequentially within described embodiments. Alternatively, a first and second agent may be administered simultaneously, and a third or additional agent may be administered before or after the first two agents.

The combination of agents used within the methods and pharmaceutical compositions described herein may have a therapeutic additive or synergistic effect on the condition(s) or disease(s) targeted for treatment. The combination of agents used within the methods or pharmaceutical compositions described herein also may reduce a detrimental effect associated with at least one of the agents when administered alone or without the other agent(s) of the particular pharmaceutical composition. For example, the toxicity of side effects of one agent may be attenuated by another agent of the composition, thus allowing a higher dosage, improving patient compliance, and improving therapeutic outcome. The additive or synergistic effects, benefits, and advantages of the compositions apply to classes of therapeutic agents, either structural or functional classes, or to individual compounds themselves.

Supplementary active compounds can also be incorporated into the compositions. In certain embodiments, an antibody or antibody portion of the invention is coformulated with and/or coadministered with one or more additional therapeutic agents that are useful for treating depression. For example, an anti-hTNFα antibody, antibody portion, may be coformulated and/or coadministered with one or more additional antibodies that bind other targets (*e*.*g*., antibodies that bind other cytokines or that bind cell surface molecules), one or more cytokines, soluble TNFα receptor (see *e.g.,* PCT Publication No. WO 94/06476) and/or one or more chemical agents that inhibit hTNFα production or activity (such as cyclohexane-ylidene derivatives as described in PCT Publication No. WO 93/19751). Furthermore, one or more antibodies of the invention may be used in combination with two or more of the foregoing therapeutic agents. Such combination therapies may advantageously utilize lower dosages of the administered therapeutic agents, thus avoiding possible toxicities or complications associated with the various monotherapies.

It should be noted that while the anti-hTNFα antibody is subcutaneously administered, where the subcutaneous administration excludes the perispinal administration for treatment of depression, the additional therapeutic agent may be administered via a different route. One of ordinary skill in the art would recognize the appropriate means by which the additional agent is administered.

The TNFα antibody for use in the invention may be used in combination with additional therapeutic agents for the treatment of depression. Additional agents used to treat depression include antidepressant agents. Examples of antidepressant agents include, but selective serotonin reuptake inhibitors (SSRIs), tricyclic antidepressants, and MAOI's (monoamine oxidase inhibitors). Examples of SSRIs include citalopram (Celexa), escitalopram oxalate (Lexapro), fluoxetine (Prozac), paroxetine (Paxil, paxil CR), and sertraline (Zoloft). Examples of tricyclic antidepressants include imipramine, amitriptyline, clomipramine, doxepin, desipramine, nortriptyline, protriptyline, and trimipramine. Examples of MAOIs include phenelzine (Nardil), tranylcypromine (Parnate), and isocarboxazid (Marplan).

Non-limiting examples of other agents that can be used in combination with a TNFα antibody for the treatment of depression or treatment of depression and an additional disorder, include but are not limited to antibodies to or antagonists of human cytokines or growth factors, for example, TNF, LT, IL-1, IL-2, IL-3, IL-4, II,-5, IL-6, IL-7, IL-8, IL-15, IL-16, IL-18, IL-21, IL-23, interferons, BMAP-II, GM-CSF, FGF, and PDGF; antibodies to cell surface molecules such as CD2, CD3, CD4, CD8, CD25, CD28, CD30, CD40, CD45, CD69, CD80 (B7.1), CD86 (B7.2), CD90, CTLA or their ligands including CD154 (gp39 or CD40L); TNFα converting enzyme (TACE) inhibitors; IL-1 inhibitors (Interleukin-1-converting enzyme inhibitors, IL-1RA etc.); Interleukin 11; IL-18 antagonists including IL-18 antibodies or soluble IL-18 receptors, or IL-18 binding proteins; non-depleting anti-CD4 inhibitors; antagonists of the co-stimulatory pathway CD80 (B7.1) or CD86 (B7.2) including antibodies, soluble receptors or antagonistic ligands; agents which interfere with signalling by proinflammatory cytokines such as TNFα or IL-1 (*e*.*g*. IRAK, NIK, IKK, p38 or MAP kinase inhibitors); IL-1β converting enzyme (ICE) inhibitors; T-cell signalling inhibitors such as kinase inhibitors; metalloproteinase inhibitors; angiotensin converting enzyme inhibitors; soluble cytokine receptors and derivatives thereof (*e*.*g*. soluble p55 or p75 TNF receptors and the derivatives p75TNFRIgG (Enbrel™ and p55TNFRIgG (Lenercept)), sIL-1RI, sIL-1RII, sIL-6R); antiinflammatory cytokines (*e*.*g*. IL-4, IL-10, IL-11, IL-13 and TGFb); Rituximab; IL-1 TRAP; MRA; CTLA4-Ig; IL-18 BP; anti-IL-18; anti-IL15; IDEC-CE9.1/SB 210396 (non-depleting primatized anti-CD4 antibody; IDEC/SmithKline; see *e.g.,* Arthritis & Rheumatism (1995) Vol. 38, S185); DAB 486-IL-2 and/or DAB 389-IL-2 (IL-2 fusion proteins; Seragen; see *e.g.,* Arthritis & Rheumatism (1993) Vol. 36, 1223); Anti-Tac (humanized anti-IL-2Ra; Protein Design Labs/Roche); IL-4 (anti-inflammatory cytokine; DNAX/Schering); IL-10 (SCH 52000; recombinant IL-10, anti-inflammatory cytokine; DNAX/Schering); IL-10 and/or IL-4 agonists *(e.g.,* agonist antibodies); IL-1RA (IL-1 receptor antagonist; Synergen/Amgen); anakinra (Kineret^{®}/Amgen); TNF-bp/s-TNF (soluble TNF binding protein; see *e.g.,* Arthritis & Rheumatism (1996) Vol. 39, No. 9 (supplement), S284; Amer. J. Physiol. - Heart and Circulatory Physiology (1995) Vol. 268, pp. 37-42); R973401 (phosphodiesterase Type IV inhibitor; see *e.g*., Arthritis & Rheumatism (1996) Vol. 39, No. 9 (supplement), S282); MK-966 (COX-2 Inhibitor; see *e.g.,* Arthritis & Rheumatism (1996) Vol. 39, No. 9 (supplement), S81); Iloprost (see *e.g.,* Arthritis & Rheumatism (1996) Vol. 39, No. 9 (supplement), S82); zap-70 and/or lck inhibitor (inhibitor of the tyrosine kinase zap-70 or Ick); VEGF inhibitor and/or VEGF-R inhibitor (inhibitors of vascular endothelial cell growth factor or vascular endothelial cell growth factor receptor; inhibitors of angiogenesis); TNF-convertase inhibitors; anti-IL-12 antibodies; anti-IL-18 antibodies; interleukin-11 (see *e.g.,* Arthritis & Rheumatism (1996) Vol. 39, No. 9 (supplement), S296); interleukin-13 (see *e.g.,* Arthritis & Rheumatism (1996) Vol. 39, No. 9 (supplement), S308); interleukin -17 inhibitors (see *e.g.,* Arthritis & Rheumatism (1996) Vol. 39, No. 9 (supplement), S120); anti-thymocyte globulin; anti-CD4 antibodies; CD5-toxins; ICAM-1 antisense phosphorothioate oligodeoxynucleotides (ISIS 2302; Isis Pharmaceuticals, Inc.); soluble complement receptor 1 (TP10; T Cell Sciences, Inc.), efalizumab, and anti-IL2R antibodies, including anti-IL12 antibody (ABT 874); anti-IL18 antibody (ABT 325); small molecule inhibitor of LCK; small molecule inhibitor of COT; anti-IL1 antibody; small molecule inhibitor of MK2; anti-CD19 antibody; small molecule inhibitor of CXCR3; small molecule inhibitor of CCR5; small molecule inhibitor of CCR11 anti-E/L selectin antibody; small molecule inhibitor of P2X7; small molecule inhibitor of IRAK-4; small molecule agonist of glucocorticoid receptor; anti-C5a receptor antibody; small molecule inhibitor of C5a receptor; anti-CD32 antibody; and CD32 as a therapeutic protein.

Examples of agents that can be used in combination with a TNFα antibody for the treatment of depression or treatment of depression and rheumatoid arthritis, include, but are not limited to, small molecule inhibitor of KDR (ABT-123), small molecule inhibitor of Tie-2; methotrexate; prednisone; celecoxib; folic acid; hydroxychloroquine sulfate; rofecoxib; etanercept; infliximab; anakinra (Kineret^{®}/Amgen); leflunomide; naproxen; valdecoxib; sulfasalazine; ibuprofen; methylprednisolone; meloxicam; methylprednisolone acetate; gold sodium thiomalate; aspirin; azathioprine; triamcinolone acetonide; propxyphene napsylate/apap; folate; nabumetone; diclofenac; piroxicam; etodolac; diclofenac sodium; oxaprozin; oxycodone hcl; hydrocodone bitartrate/apap; diclofenac sodium/misoprostol; fentanyl; anakinra, human recombinant; tramadol hcl; salsalate; sulindac; cyanocobalamin/fa/ pyridoxine; acetaminophen; alendronate sodium; prednisolone; morphine sulfate; lidocaine hydrochloride; indomethacin; glucosamine sulfate/chondroitin; cyclosporine; sulfadiazine; amitriptyline hcl; oxycodone hcl/acetaminophen; olopatadine hcl; misoprostol; naproxen sodium; omeprazole; mycophenolate mofetil; cyclophosphamide; rituximab; IL-1 TRAP; MRA; CTLA4-IG; IL-18 BP; ABT-874; ABT-325 (anti-IL 18); anti-IL 15; BIRB-796; SCIO-469; VX-702; AMG-548; VX-740; Roflumilast; IC-485; CDC-801; and mesopram.

In yet another embodiment, a TNFα antibody may be administered for the treatment of depression or treatment of depression and an additional disorder, in combination with an antibiotic or antiinfective agent. Antiinfective agents include those agents known in the art to treat viral, fungal, parasitic or bacterial infections. The term, "antibiotic," as used herein, refers to a chemical substance that inhibits the growth of, or kills, microorganisms. Encompassed by this term are antibiotic produced by a microorganism, as well as synthetic antibiotics (*e.g*., analogs) known in the art. Antibiotics include, but are not limited to, clarithromycin (Biaxin^{®}), ciprofloxacin (Cipro^{®}), and metronidazole (Flagyl^{®}).

The invention also includes administration of a TNFα antibody for the treatment of depression or treatment of depression and an additional disorder, in combination with a drug used to treat Crohn's disease or a Crohn's-related disorder. Examples of therapeutic agents which can be used to treat Crohn's disease include mesalamine, prednisone, azathioprine, mercaptopurine, infliximab, budesonide, sulfasalazine, methylprednisolone sod succ, diphenoxylate/atrop sulf, loperamide hydrochloride, methotrexate, omeprazole, folate, ciprofloxacin/ dextrose-water, hydrocodone bitartrate/apap, tetracycline hydrochloride, fluocinonide, metronidazole, thimerosal/boric acid, hyoscyamine sulfate, cholestyramine/sucrose, ciprofloxacin hydrochloride, meperidine hydrochloride, midazolam hydrochloride, oxycodone hcl/acetaminophen, promethazine hydrochloride, sodium phosphate, sulfamethoxazole/trimethoprim, celecoxib, polycarbophil, propoxyphene napsylate, hydrocortisone, multivitamins, balsalazide disodium, codeine phosphate/apap, colesevelam hcl, cyanocobalamin, folic acid, levofloxacin, natalizumab, methylprednisolone, interferon-gamma, and sargramostim (GM-CSF). In one embodiment, methotrexate is administered for the treatment of Crohn's disease at a dose of 2.5 mg to 30 mg per week.

In another embodiment, the invention includes administration of a TNFα antibody for the treatment of depression or treatment of depression and an additional disorder, wherein the additional disorder is a spondyloarthropathy. The invention includes the administration of a TNF inhibitor in combination with a drug used to treat spondyloarthropathies. Examples of such agents include nonsteroidal, anti-inflammatory drugs (NSAIDs), COX 2 inhibitors, including Celebrex^{®}, Vioxx^{®}, and Bextra^{®}, aand etoricoxib. Physiotherapy is also commonly used to treat spondyloarthropathies, usually in conjunction with non-steoidal inflammatory drugs.

In another embodiment, non-limiting examples of agents that can be used in a combination treatment with a TNFα antibody for the treatment of depression or treatment of depression and ankylosing spondylitis, include but are not limited to ibuprofen, diclofenac and misoprostol, naproxen, meloxicam, indomethacin, diclofenac, celecoxib, rofecoxib, sulfasalazine, prednisone, methotrexate, azathioprine, minocyclin, prednisone, etanercept, and infliximab.

In another embodiment, the TNFα antibody of the invention is administered in combination with an additional therapeutic agent to treat psoriatic arthritis and depression. Examples of agents which can be used to reduce or inhibit the symptoms of psoriatic arthritis include methotrexate; etanercept; rofecoxib; celecoxib; folic acid; sulfasalazine; naproxen; leflunomide; methylprednisolone acetate; indomethacin; hydroxychloroquine sulfate; sulindac; prednisone; betamethasone diprop augmented; infliximab; methotrexate; folate; triamcinolone acetonide; diclofenac; dimethylsulfoxide; piroxicam; diclofenac sodium; ketoprofen; meloxicam; prednisone; methylprednisolone; nabumetone; tolmetin sodium; calcipotriene; cyclosporine; diclofenac; sodium/misoprostol; fluocinonide; glucosamine sulfate; gold sodium thiomalate; hydrocodone; bitartrate/apap; ibuprofen; risedronate sodium; sulfadiazine; thioguanine; valdecoxib; alefacept; and RAPTTVA® (efalizumab).

The TNFα antibody may be administered in combination with topical corticosteroids, vitamin D analogs, and topical or oral retinoids, or combinations thereof, for the treatment of psoriasis. In addition, the TNFα antibody may be administered in combination with one of the following agents for the treatment of psoriasis: small molecule inhibitor of KDR (ABT-123), small molecule inhibitor of Tie-2, calcipotriene, clobetasol propionate, triamcinolone acetonide, halobetasol propionate, tazarotene, methotrexate, fluocinonide, betamethasone diprop augmented, fluocinolone, acetonide, acitretin, tar shampoo, betamethasone valerate, mometasone furoate, ketoconazole, pramoxine/fluocinolone, hydrocortisone valerate, flurandrenolide, urea, betamethasone, clobetasol propionate%moll, fluticasone propionate, azithromycin, hydrocortisone, moisturizing formula, folic acid, desonide, coal tar, diflorasone diacetate, etanercept, folate, lactic acid, methoxsalen, hc/bismuth subgal/znox/resor, methylprednisolone acetate, prednisone, sunscreen, salicylic acid, halcinonide, anthralin,
clocortolone pivalate, coal extract, coal tar/salicylic acid, coal tar/salicylic acid/sulfur, desoximetasone, diazepam, emollient, pimecrolimus emollient, fluocinonide/emollient, mineral oil/castor oil/na lact, mineral oil/peanut oil, petroleum/isopropyl myristate, psoralen, salicylic acid, soap/tribromsalan, thimerosal/boric acid, celecoxib, infliximab, alefacept, RAPTIVA® (efalizumab), tacrolimus, pimecrolimus, PUVA, UVB and other phototherapy, and sulfasalazine.

An antibody, antibody portion, may be used in combination with other agents to treat skin conditions in subjects also having depression. For example, an antibody, antibody portion, or other TNFα inhibitor of the invention is combined with PUVA therapy. PUVA is a combination of psoralen (P) and long-wave ultraviolet radiation (UVA) that is used to treat many different skin conditions. The antibodies, antibody portions, or other TNFα inhibitors of the invention can also be combined with pimecrolimus. In another embodiment, the antibodies of the invention are used to treat psoriasis, wherein the antibodies are administered in combination with tacrolimus. In a further embodiment, tacrolimus and TNFα inhibitors are administered in combination with methotrexate and/or cyclosporine. In still another embodiment, the TNFα inhibitor of the invention is administered with excimer laser treatment for treating psoriasis and depression.

Any one of the above-mentioned therapeutic agents, alone or in combination therewith, can be administered to a subject suffering from depression or to a subject suffering from depression and an additional disorder, in combination with the TNFα antibody. Furthermore, the additional agents described for specific additional disorders are not limited to said disorders, but may be used in a combination therapy with aTNFα antibody for treatment of depression or depression and an additional disorder.

### EXAMPLES

### Example 1: Systemic administration of a TNFα antibody treats depression using a murine model

The following example demonstrates the effect of a murine antibody as an inhibitor against TNFα in the depressive-like behaviors elicited following the systemic administration of recombinant mouse TNF-α in mouse Tail Suspension Test. The mouse Tail Suspension Test (mTST) is one of the most extensively used animal models of depression, and is considered to have a good predictive value for detecting antidepressant activity. The model is based on the experimental procedure described by Steru et al. Psychopharmacology (1985) 85:367-370 and Steru et al. Prog. Neuro-Psychopharmacol. & Biol. Psychiat. (1987) 11:659-671). The test assesses immobility time in animals exposed to a short-term inescapable stressor (being suspended by the tail). After initial vigorous attempts to struggle and escape from the situation, the mice develop an immobile posture, which has been called behavioral despair, suggestive of a "depressive-like state", and hypothesized to reflect lowered mood or hopelessness. TST is sensitive to a broad variety of antidepressant treatments, which significantly reduce the immobility period and promote the occurrence of escape-oriented behaviors in the test.

For the procedure, animals were habituated to the testing room for approximately 1-1.5h before starting the experiment. Following the drug treatment time, a piece of tape was wrapped around the tail 20 mm from the tip. The mouse was hung by the tape from a hook attached to a transducer, which communicated information about duration of movements to a computer. The test took 6 min, during which immobility time (s) was recorded.

A previous experiment showed that systemic administration of recombinant mouse TNF-α (rmTNF-α, 5µg/mouse, i.p.) induced depressive-like behavior and increased immobility time in mTST when mice were tested 1h after administration, compared to a control group of mice treated with phosphate buffered saline (PBS) solution (p<0.05). In order to better understand the role of this proinflammatory cytokine in mood disorders and the potential use of TNF-α antibodies for major depression, the aim of this experiment was to investigate the effects of the murine anti- TNF-α antibody administered systemically (i.p.), in depressive-like behaviors elicited by peripheral administration of rmTNF-α.

Male Balb/c mice weighing about 20-25g (Charles River Laboratories) were used for the experiment. After habituation to the testing room, animals were assigned to two groups that were injected i.p. with either mouse Immunoglobulin G (IgG, 300 µg/mice) (Sigma, St. Louis, MO) or the murine antibody against TNF-α (ABC, Abbott Laboratories) (300 µg/mice), both dissolved in PBS solution administered in a volume of 0.25 ml/ mouse. Two hours after the pretreatment, half of the animals belonging to each experimental group received a second systemic administration (i.p. injection) of the rmTNF-α (E. coli-derived, 5 µg/0.25 ml/mouse) (R&D Systems, Minneapolis, MN) and the remaining mice received an injection of PBS solution (0.25 ml). Behavioral testing in the mTST took place 1 hour after the second administration.

Data were analyzed using a two-way ANOVA where the main factors under consideration were pretreatment (IgG or mouse TNF-α antibody) and TNF-α treatment (PBS or rmTNF-α). Post-hoc analysis for individual group comparisons was done using Fisher's protected least significant difference test.

A two-way ANOVA revealed a significant pretreatment effect (antibody) (F₁,₃₆= 8.81, p<0.01), a significant effect of rmTNF-α treatment (F₁,₃₆= 20.02, p<0.001) and a significant pretreatment x treatment interaction (F₁,₃₆= 26.7, p<0.001). rmTNF-α i.p. induced depressive-like behavior as demonstrated by increased immobility time in the mTST compared to the appropriate control group (IgG + TNF-α compared to IgG + PBS) (p<0.001). Systemic injection of murine monoclonal antibody against TNF-α, but not the administration of IgG (control group), blocked the depressive-like behavior induced by rmTNF-α administration. In summary, depressive-like behaviors induced by rmTNF-α were antagonized by systemic administration of the monoclonal antibody against rmTNF-α. These results support a role for systemic/ peripheral action of rmTNF-a in depression and the potential use of TNFα antibody for the treatment of depressive disorders.

**Table 1: Effect of murine monoclonal antibody against TNF-α in depressive-like behaviors elicited following the systemic administration of rmTNF-α in mTST. Values represent immobility time (s) during the 6-min test in the mTST model of depression.**

| Pretreatment | IgG | | Anti TNF-α | |
|---|---|---|---|---|
| Treatment | PBS | rmTNF-α | PBS | rmTNF-α |
| Mean ± SEM | 139.1 ± 25.16 | 321.5 ±7.97 *** | 180.7 ± 21.40 | 167.6 ± 16.64 ### |

| | | | | |
|---|---|---|---|---|
| Data are expressed as Mean ± Standard Error of the Mean (SEM). N=10 in each experimental group ***p<0.001 compared to IgG + PBS group, ###p<0001 compared to IgG + TNF-α | | | | |

### Example 2: A Randomized, Double-Blind, Placebo-Controlled Study of the Efficacy and Safety of TNFα Antibody in Treatment-Resistant Depression (TRD).

The objective of this study is to explore the efficacy and safety of a TNFα antibody, *i.e.,* adalimumab, compared to a placebo during a 9-week adjunctive therapy in treatment-resistant depression (TRD) patients.

To determine the efficacy and safety of adalimumab in TRD patients, a 9-week, randomized, double-blind, parallel group, multi-center study is conducted to explore the efficacy and safety of adalimumab compared to placebo as adjunctive therapy. The study is conducted in approximately 120 patients diagnosed with major depression and having a HAM- D₂₄ score ≥ 20, despite two previous adequate treatment trials with standard anti-depressant medications. The study consists of a screening period of approximately 7 days and a 9-week treatment period. Subjects are randomly assigned to each of the two treatment groups (adalimumab 40mg once weekly (QW) and matching placebo) in equal numbers with approximately 60 subjects per group.

The main inclusion criteria include the following: male or non-pregnant, non-lactating female (female subjects of childbearing potential must be using effective contraception); age between 18 and 75, inclusive, at the time of randomization; a current DSM-IV-TM primary diagnosis of major unipolar depression without psychotic features as confirmed by the Structured Clinical Interview for DSM-IV-TR (SCID); satisfaction of the criteria for Treatment-Resistant Depression as defined by a HAM-D₂₄ scope ≥2:20 despite two previous adequate treatment trials with standard anti-depressant medications; a stable anti-depressant regimen for ≥ 4 weeks; general good health; and informed consent.

Exclusion criteria, considered alone or in combination, include: history of cancer or lymphoproliferative disease; history of listeria, HIV, Hepatitis B or C, immunodeficiency, demyelinating disease or tuberculosis; poorly controlled medical condition, or any condition for which surgery or medication change is anticipated during study; recent systemic antibiotic, antiviral or antifungal treatment; positive C. difficile stool assay; hospitalized, in immediate need of hospitalization or has been repeatedly hospitalized because of current depression episode; diagnosis of bipolar disorder or personality disorder; serious suicidal ideation in the opinion of the investigator; recent, clinically significant drug or alcohol abuse; positive urine drug or alcohol screen; prior use of any anti-TNF agent; hypersensitivity to excipients of adalimumab as stated in the label; use of other immune modifying medications; and recent receipt of investigational agent or device.

Patients receive adalimumab, administered at a dose of 40mg with a device, *e.g.,* a pen device. The mode of systemic administration is subcutaneous injection, excluding perispinal injection. The reference therapy is a placebo for adalimumab, systemically administered with a device, *e.g*., a pen device, for example, by subcutaneous injection. The duration of the study is 9 weeks, with one week double-blind placebo lead-in and 8 weeks double-blind treatment.

The criteria for evaluation of adalimumab for the treatment of major depression include efficacy, pharmacodynamic, and safety. The primary efficacy variable is the HAM-D total score change from baseline to last study visit (day 64). Other efficacy variables used to determine the efficacy of adalimumab for the treatment of major depression include HAM-A, CGI, PGI, and quality of life scores. With regard to phamacodynamic evaluation, biomarkers including DNA, cytokines, metabolites and sleep quality can be explored as predictors of response. A plasma sample can be banked for potential additional exploratory analyses. Safety measures include vital signs, previous concomitant medication, anti-nuclear antibodies (ANA), electrocardiogram (ECG), urine toxicology, urine pregnancy, hematology/general labs, urinalysis and adverse events (AE) monitoring.

Treatment of depression is indicated by an improvement or remission response in the patient as indicated by the HAM-D score. Statistical methods in efficacy are conducted by a maximum likelihood-based, mixed-model repeated measures analysis of HAM-D total score. The model includes the fixed categorical effects of treatment, investigator, visit and treatment-by-visit interaction and the continuous fixed covarities of screening HAM-D total score and screening-by-visit interaction. The primary comparison is the contrast on change from screening to the last visit of the study. Additionally, HAM-D change from baseline to the last observation is assessed using analysis of covariance with factors for treatment and investigator, and the screening HAM-D total score as a covariate. These analyses are also performed for the other efficacy variables. Response (HAM-D decrease ≥ 50%) and remission (HAM-D ≤ 7) rates at each visit and at the last observation are assessed using Fisher's exact test, and are used to determine the efficacy of adalimumab for improving depression in a patient.

Exploratory biomarker analyses are also performed. Descriptive safety statistics are tabulated by treatment group of changes from baseline to last observation for continuous variables and incidence rates for categorical variables. Continuous variables are assessed using analysis of variance with factors for treatment, investigator, and treatment-by-investigator interaction. As appropriate, categorical variables are assessed using Fisher's exact test

### EQUIVALENTS

Those skilled in the art will recognize, or be able to ascertain using no more than routine experimentation, many equivalents to the specific embodiments of the invention described herein. Such equivalents are intended to be encompassed by the following claims.

## Claims

1. An isolated human anti-TNFα antibody, or an antigen binding portion thereof, for use in the treatment of depression, wherein the human anti-TNFα antibody, or the antigen binding portion thereof, is subcutaneously administered to the subject and wherein the subcutaneous administration excludes perispinal administration.

2. The human anti-TNFα antibody, or antigen-binding portion thereof, of claim 1, for the use in the treatment of depression, which dissociates from human TNFα with a Kd of 1 x 10-8M or less and a Koff rate constant of 1 x 10-3 s-1 or less, both determined by surface plasmon resonance, and neutralizes human TNFα cytotoxicity in a standard *in vitro* L929 assay with an IC50 of 1 x 10-7 M or less.

3. The human anti-TNFα antibody, or antigen-binding portion thereof, of claim 1, for the use in the treatment of depression, which has the following characteristics:
a) dissociates from human TNFα with a Koff rate constant of 1 x 10-3 s-1 or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9; and
c) has a heavy chain CDR3 domain comprising the amino acid sequence
of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3,4, 5, 6, 8, 9, 10, 11 and/or 12.

4. The human anti-TNFα antibody, or antigen-binding portion thereof, of claim 1, for the use in the treatment of depression, which comprises a light chain variable region (LCVR) comprising the amino acid sequence of SEQ ID NO: 1 and a heavy chain variable region (HCVR) comprising the amino acid sequence of SEQ ID NO: 2

5. The human anti-TNFα antibody, or antigen-binding portion thereof, of claim 1, for the use in the treatment of depression, which is adalimumab (D2E7) or golimumab.

6. The human anti-TNFα antibody, or antigen-binding portion thereof, of any one of claims 1-5, for the use in the treatment of depression, wherein the depression is selected from the group consisting of major depression, dysthmic disorder, bipolar disorder I, bipolar disorder II, and cyclothymic disorder.

7. The human anti-TNFα antibody, or antigen-binding portion thereof, of claim 6, for the use in the treatment of depression, wherein the depression is major depression and is either a single episode, recurrent, refractory or treatment resistant depression.

8. The human anti-TNFα antibody, or antigen-binding portion thereof, of claim 6, for the use in the treatment of depression, wherein the depression is either dysthmic disorder, bipolar disorder I, or bipolar disorder II and occurs in combination with catatonic features, melancholic features, or with atypical features of postpartum depression.

9. The human anti-TNFα antibody, or antigen-binding portion thereof, of any one claims 1-5, for the use in the treatment of depression, wherein the subject has an additional disorder selected from the group consisting of a TNFa-related disorder, coronary heart disease, neurodegenerative disease, stroke, infectious disease, autoimmune disorder, psoriasis, psoriatic arthritis, rheumatoid arthritis, ankylosing spondylitis, juvenile idiopathic arthritis, juvenile rheumatoid arthritis, undifferentiated spondyloarthopathies, intestinal disorder, inflammatory bowel disease, Crohn's disease, Behcet's disease, asthma, and Niemann-Pick disease.

10. The human anti-TNFα antibody, or antigen-binding portion thereof, of any one of claims 1-5, for the use in the treatment of depression, wherein the treatment of depression further comprises administering an antidepressant agent.

11. The human anti-TNFα antibody, or antigen-binding portion thereof, of any one of claims 1-5, for the use in the treatment of depression, wherein the antibody is administered on a dosing regimen selected from the group consisting of a biweekly dosing regimen, a multiple variable dose regimen, and a weekly dosing regimen.

12. The human anti-TNFα antibody, or antigen-binding portion thereof, of any one of claims 1-5, for the use in the treatment of depression, wherein the antibody is administered in a 40 mg dose.

## Patentansprüche

1. Ein isolierter menschlicher anti-TNFα-Antikörper, oder ein Antigen-bindender Abschnitt davon, für die Verwendung in der Behandlung von Depression, worin der menschliche anti-TNFα-Antikörper oder der Antigen-bindende Abschnitt davon subkutan an das Subjekt verabreicht wird, und worin die subkutane Verabreichung die perispinale Verabreichung ausschließt.

2. Der menschliche anti-TNFα-Antikörper oder der Antigen-bindende Abschnitt davon, gemäß Anspruch 1, für die Verwendung in der Behandlung von Depression, welcher von menschlichem TNFα dissoziiert mit einer Kd von 1 x 10⁻⁸ M oder weniger und einer K_{off} Geschwindigkeitskonstante von 1 x 10⁻³ s⁻¹ oder weniger, beides bestimmt durch Oberflächenplasmonresonanz, und welcher menschliche TNFα Cytotoxizität neutralisiert in einem Standard *in vitro* L929 Assay mit einer IC50 von 1 x 10⁻⁷ M oder weniger.

3. Der menschliche anti-TNFα-Antikörper oder der Antigen-bindende Abschnitt davon gemäß Anspruch 1, für die Verwendung in der Behandlung von Depression, welcher die folgenden Charakteristika hat:
a) dissoziiert von menschlichem TNFα mit einer K_{off} Geschwindigkeitskonstante von 1 x 10⁻³ s⁻¹ oder weniger, wie bestimmt durch Oberflächenplasmonresonanz;
b) hat eine leichte Kette CDR3 Domain umfassend die Aminosäuresequenz von Sequenzidentifikationsr.: 3, oder modifiziert von Sequenzidentifikationsnr.: 3 durch eine einzelne Alaninsubstitution an Position 1, 4, 5, 7 oder 8, oder durch eine bis fünf konservative Aminosäuresubstitutionen an Positionen 1, 3, 4, 6, 7, 8 und/oder 9; und
c) hat eine schwere Kette CDR3 Domain umfassend die Aminosäuresequenz von Sequenzidentifikationsnr.: 4, oder modifiziert von Sequenzidentifikationsnr.: 4 durch eine einzelne Alaninsubstitution an Position 2, 3, 4, 5, 6, 8, 9, 10 oder 11 oder durch eine bis fünf konservative Aminosäuresubstitutionen an Positionen 2, 3, 4, 5, 6, 8, 9, 10, 11 und/oder 12.

4. Der menschliche anti-TNFα-Antikörper oder der Antigenbindende Abschnitt davon gemäß Anspruch 1, für die Verwendung in der Behandlung von Depression, welcher eine leichte Kette variable Region (LCVR) umfassend die Aminosäuresequenz von Sequenzidentifikationsnr.: 1 und eine schwere Kette variable Region (HCVR) umfassend die Aminosäuresequenz der Sequenzidentifikationsnr.: 2 umfasst.

5. Der menschliche anti-TNFα-Antikörper oder der Antigen-bindende Abschnitt davon gemäß Anspruch 1, für die Verwendung in der Behandlung von Depression, welcher Adalimumab (D2E7) oder Golimumab ist.

6. Der menschliche anti-TNFα-Antikörper oder der Antigen-bindende Abschnitt davon gemäß irgendeinem der Ansprüche 1-5, für die Verwendung in der Behandlung von Depression, worin die Depression gewählt ist aus der Gruppe bestehend aus klinischer Depression, dysthymischer Störung, bipolarer Störung I, bipolarer Störung II, und cyclothymischer Störung.

7. Der menschliche anti-TNFα-Antikörper oder der Antigenbindende Abschnitt davon gemäß Anspruch 6, für die Verwendung in der Behandlung von Depression, worin die Depression klinische Depression ist und entweder eine Einzelepisodenwiederkehrende, beständige oder Behandlungs-resistente Depression ist.

8. Der menschliche anti-TNFα-Antikörper oder der Antigen-bindende Abschnitt davon gemäß Anspruch 6, für die Verwendung in der Behandlung von Depression, worin die Depression entweder dysthymische Störung, bipolare Störung I oder bipolare Störung II ist, und in Kombination mit katatonischen Merkmalen, melancholischen Merkmalen, oder mit atypischen Merkmalen der Wochenbettdepression auftritt.

9. Der menschliche anti-TNFα-Antikörper oder der Antigen-bindende Abschnitt davon gemäß irgendeinem der Ansprüche 1 bis 5, für die Verwendung in der Behandlung von Depression, worin das Subjekt eine zusätzliche Krankheit hat gewählt aus der Gruppe bestehend aus einer TNFα-verwandten Erkrankung, koronarer Herzkrankheit, neurodegenerativer Erkrankung, Schlaganfall, infektiöser Erkrankung, Autoimmunerkrankung, Psoriasis, psoriatischer Arthritis, rheumatoider Arthritis, Morbus Bechterew, juveniler idiopathischer Arthritis, juveniler rheumatoider Arthritis, undifferenzierten Spondyloarthropathien, Darmerkrankung, entzündlicher Darmerkrankung, Morbus Crohn, Morbus Behcet, Asthma und Niemann-Pick Krankheit.

10. Der menschliche anti-TNFα-Antikörper oder der Antigen-bindende Abschnitt davon gemäß irgendeinem der Ansprüche 1-5, für die Verwendung in der Behandlung von Depression, worin die Behandlung von Depression weiter das Verabreichen eines Antidepressivums umfasst.

11. Der menschliche anti-TNFα-Antikörper oder der Antigen-bindende Abschnitt davon gemäß irgendeinem der Ansprüche 1-5, für die Verwendung in der Behandlung von Depression, worin der Antikörper in einem Dosisregime gewählt aus der Gruppe bestehend aus einem zweiwöchigem Dosisregime, einem multiplen variablen Dosisregime und einem wöchentlichen Dosisregime, verabreicht wird,

12. Der menschliche anti-TNFα-Antikörper oder der Antigen-bindende Abschnitt davon gemäß irgendeinem der Ansprüche 1-5, für die Verwendung in der Behandlung von Depression, worin der Antikörper in einer 40 mg Dosis verabreicht wird.

## Revendications

1. Anticorps anti-TNFα humain isolé, ou partie de liaison d'antigène de celui-ci, destiné à être utilisé dans le traitement de la dépression, où l'anticorps anti-TNFα humain, ou sa partie de liaison d'antigène, est administré par vole sous-cutanée au sujet et où l'administration sous-cutanée exclut l'administration périspinale.

2. Anticorps anti-TNFα humain, ou partie de liaison d'antigène de celui-ci, selon la revendication 1, destiné à être utilisé dans le traitement de la dépression, qui se dissocie du TNFα humain avec une Kd de 1 x 10-8M ou moins et une constante de vitesse Koff de 1 x 10-3 s-1 ou moins, l'une et l'autre déterminées par résonance de plasmon de surface, et neutralise la cytotoxicité de TNFα humain dans un test sur L929 *in vitro* standard avec une IC50 de 1 x 10-7 M ou moins,

3. Anticorps anti-TNFα humain, ou partie de liaison d'antigène de celui-ci, selon la revendication 1, destiné à être utilisé dans le traitement de la dépression, qui a les caractéristiques suivantes ;
a) se dissocie de TNFα humain avec une constante de vitesse Koff de 1 x 10-3 s-1 ou moins, comme déterminé par résonance de plasmon de surface ;
b) a une domaine CDR3 de chaîne légère comprenant la séquence d'acides aminés de SEQ ID NO:3, ou modifiée à partir de SEQ ID NO:3 par une substitution d'alanine unique à la position 1, 4, 5, 7 ou 8 ou par une à cinq substitutions d'acides aminés conservatrices aux positions 1, 3, 4, 6, 7, 8 et/ou 9 ; et
c) a un domaine CDR3 de chaîne lourde comprenant la séquence d'acides aminés de SEQ ID NO:4 ou modifiée à partir de SEQ ID NO:4 par une substitution d'alanine unique à la position 2, 3, 4, 5, 6, 8, 9, 10 ou 11 ou par une à cinq substitutions d'acides aminés conservatrices aux positions 2, 3, 4, 5, 6, 8, 9, 10, 11 et/ou 12.

4. Anticorps anti-TNFα humain, ou partie de liaison d'antigène de celui-ci, selon la revendication 1, destiné à être utilisé dans le traitement de la dépression, qui comprend une région variable de chaîne légère (LCVR) comprenant la séquence d'acides aminés de SEQ ID NO:1 et une région variable de chaîne lourde (HCVR) comprenant la séquence d'acides aminés de SEQ ID NO:2.

5. Anticorps anti-TNFα humain, ou partie de liaison d'antigène de celui-ci, selon la revendication 1, destiné à être utilisé dans le traitement de la dépression, qui est l'adalimumab (D2E7) ou le golimumab.

6. Anticorps anti-TNFα humain, ou partie de liaison d'antigène de celui-ci, selon l'une quelconque des revendications 1-5, destiné à être utilisé dans le traitement de la dépression, où la dépression est choisie dans le groupe consistant en la dépression majeure, le trouble dysthymique, le trouble bipolaire I, le trouble bipolaire II et le trouble cyclothymique.

7. Anticorps anti-TNFα humain, ou partie de liaison d'antigène de celui-ci, selon la revendication 6, destiné à être utilisé dans le traitement de la dépression, où la dépression est la dépression majeure et est une dépression à un seul épisode, récurrente, réfractaire ou résistante aux traitements.

8. Anticorps anti-TNFα humain, ou partie de liaison d'antigène de celui-ci, selon la revendication 6, destiné à être utilisé dans le traitement de la dépression, où la dépression est le trouble dysthymique, le trouble bipolaire 1 ou le trouble bipolaire II et survient en combinaison avec des aspects catatoniques, des aspects mélancoliques ou avec des aspects atypiques de dépression postpartum.

9. Anticorps anti-TNFα humain, ou partie de liaison d'antigène de celui-ci, selon l'une quelconque des revendications 1-5, destiné à être utilisé dans le traitement de la dépression, où le sujet a un trouble supplémentaire choisi dans le groupe consistant en un trouble lié à TNFa, une maladie coronarienne, une maladie neurodégénérative, une attaque, une maladie infectieuse, un trouble auto-immun, le psoriasis, le rhumatisme psoriasique, la polyarthrite rhumatoïde, la spondylarthrite ankylosante, l'arthrite idiopathique juvénile, la polyarthrite rhumatoïde juvénile, les spondyloarthropathies non différenciées, un trouble intestinal, une maladie intestinale inflammatoire, la maladie de Crohn, la maladie de Behcet, l'asthme et la maladie de Nlemann-Pick.

10. Anticorps anti-TNFα humain, ou partie de liaison d'antigène de celui-ci, selon l'une quelconque des revendications 1-5, destiné à être utilisé dans le traitement de la dépression, où le traitement de la dépression comprend en outre l'administration d'un agent antidépresseur.

11. Anticorps anti-TNFα humain, ou partie de liaison d'antigène de celui-ci, selon l'une quelconque des revendications 1-5, destiné à être utilisé dans le traitement de la dépression, où l'anticorps est administré selon un schéma posologique choisi dans le groupe consistant en un schéma posologique semihebdomadaire, un schéma à doses variables multiples et un schéma posologique hebdomadaire.

12. Anticorps anti-TNFα humain, ou partie de liaison d'antigène de celui-ci, selon l'une quelconque des revendications 1-5, destiné à être utilisé dans le traitement de la dépression, où l'anticorps est administré à une dose de 40 mg.


### SEQUENCE LISTING

(1) INFORMATION FOR SEQ ID NO:1:
   (i) SEQUENCE CHARACTERISTICS:
      (A) LENGTH: 107 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 121 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Modified-site
      (B) LOCATION: 9
      (D) OTHER INFORMATION: /note= "Xaa is Thr or Ala"
   (xi) SEQUENCE DESCRIPTION: SEQ ID NO:3:
   Gln Arg Tyr Asn Arg Ala Pro Tyr Xaa
   1               5
(2) INFORMATION FOR SEQ ID NO:4:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide (v) FRAGMENT TYPE: internal
   (ix) FEATURE:
      (A) NAME/KEY: Modified-site
      (B) LOCATION: 12
      (D) OTHER INFORMATION: /note= "Xaa is Tyr or Asn"
   (xi) SEQUENCE DESCRIPTION: SEQ ID NO:4:
   Val Ser Tyr Leu Ser Thr Ala Ser Ser Leu Asp Xaa
   1               5                   10
(2) INFORMATION FOR SEQ ID NO:5:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:5:
   Ala Ala Ser Thr Leu Gln Ser
   1               5
(2) INFORMATION FOR SEQ ID NO:6:
   (i) SEQUENCE CHARACTERISTICS:
      (A) LENGTH: 17 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:6:
   Ala Ile Thr Trp Asn Ser Gly His Ile Asp Tyr Ala Asp Ser Val Glu
   1               5                   10                  15
   Gly
(2) INFORMATION FOR SEQ ID NO:7:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:7:
   Arg Ala Ser Gln Gly Ile Arg Asn Tyr Leu Ala
   1               5                   10
(2) INFORMATION FOR SEQ ID NO:8:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:8:
   Asp Tyr Ala Met His
   1               5
(2) INFORMATION FOR SEQ ID NO:9:
   (i) SEQUENCE CHARACTERISTICS:
      (A) LENGTH: 107 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:9:
   Asp Ile Gln Met Thr Gln Ser Pro Ser Ser Leu Ser Ala Ser Ile Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Lys Tyr Asn Ser Ala Pro Tyr
                   85                  90                  95
   Ala Phe Gly Gln Gly Thr Lys Val Glu Ile Lys
               100                 105
(2) INFORMATION FOR SEQ ID NO:10:
   (i) SEQUENCE CHARACTERISTICS:
      (A) LENGTH: 121 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:10:
   Gln Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Asp Trp Val
           35                  40                  45
   Ser Ala Ile Thr Trp Asn Ser Gly His Ile Asp Tyr Ala Asp Ser Val
       50                  55                  60
   Glu Gly Arg Phe Ala Val Ser Arg Asp Asn Ala Lys Asn Ala Leu Tyr
   65                  70                  75                  80
   Leu Gln Met Asn Ser Leu Arg Pro Glu Asp Thr Ala Val Tyr Tyr Cys
                   85                  90                  95
   Thr Lys Ala Ser Tyr Leu Ser Thr Ser Ser Ser Leu Asp Asn Trp Gly
               100                 105                 110
   Gln Gly Thr Leu Val Thr Val Ser Ser
           115                 120
(2) INFORMATION FOR SEQ ID NO:11:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:11:
   Gln Lys Tyr Asn Ser Ala Pro Tyr Ala
   1               5
(2) INFORMATION FOR SEQ ID NO:12:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:12:
   Gln Lys Tyr Asn Arg Ala Pro Tyr Ala
   1               5
(2) INFORMATION FOR SEQ ID NO:13:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:13:
   Gln Lys Tyr Gln Arg Ala Pro Tyr Thr
   1               5
(2) INFORMATION FOR SEQ ID NO:14:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:14:
   Gln Lys Tyr Ser Ser Ala Pro Tyr Thr
   1               5
(2) INFORMATION FOR SEQ ID NO:15:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:15:
   Gln Lys Tyr Asn Ser Ala Pro Tyr Thr
   1               5
(2) INFORMATION FOR SEQ ID NO:16:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:16:
   Gln Lys Tyr Asn Arg Ala Pro Tyr Thr
   1               5
(2) INFORMATION FOR SEQ ID NO:17:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:17:
   Gln Lys Tyr Asn Ser Ala Pro Tyr Tyr
   1               5
(2) INFORMATION FOR SEQ ID NO:18:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:18:
   Gln Lys Tyr Asn Ser Ala Pro Tyr Asn
   1               5
(2) INFORMATION FOR SEQ ID NO:19:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:19:
   Gln Lys Tyr Thr Ser Ala Pro Tyr Thr
   1               5
(2) INFORMATION FOR SEQ ID NO:20:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:20:
   Gln Lys Tyr Asn Arg Ala Pro Tyr Asn
   1               5
(2) INFORMATION FOR SEQ ID NO:21:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:21:
   Gln Lys Tyr Asn Ser Ala Ala Tyr Ser
   1               5
(2) INFORMATION FOR SEQ ID NO:22:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:22:
   Gln Gln Tyr Asn Ser Ala Pro Asp Thr
   1               5
(2) INFORMATION FOR SEQ ID NO:23:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:23:
   Gln Lys Tyr Asn Ser Asp Pro Tyr Thr
   1               5
(2) INFORMATION FOR SEQ ID NO:24:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:24:
   Gln Lys Tyr Ile Ser Ala Pro Tyr Thr
   1               5
(2) INFORMATION FOR SEQ ID NO:25:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:25:
   Gln Lys Tyr Asn Arg Pro Pro Tyr Thr
             1                   5
(2) INFORMATION FOR SEQ ID NO:26:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:26:
   Gln Arg Tyr Asn Arg Ala Pro Tyr Ala
   1               5
(2) INFORMATION FOR SEQ ID NO:27:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:27:
   Ala Ser Tyr Leu Ser Thr Ser Ser Ser Leu Asp Asn
   1               5                   10
(2) INFORMATION FOR SEQ ID NO:28:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:28:
   Ala Ser Tyr Leu Ser Thr Ser Ser Ser Leu Asp Lys
   1               5                   10
(2) INFORMATION FOR SEQ ID NO:29:
   (i) SEQUENCE-CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:29:
   Ala Ser Tyr Leu Ser Thr Ser Ser Ser Leu Asp Tyr
   1               5                   10
(2) INFORMATION FOR SEQ ID NO:30:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:30:
   Ala Ser Tyr Leu Ser Thr Ser Ser Ser Leu Asp Asp
   1               5                   10
(2) INFORMATION FOR SEQ ID NO:31:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:31:
   Ala Ser Tyr Leu Ser Thr Ser Phe Ser Leu Asp Tyr
   1               5                   10
(2) INFORMATION FOR SEQ ID NO:32:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:32:
   Ala Ser Tyr Leu Ser Thr Ser Ser Ser Leu His Tyr
   1               5                   10
(2) INFORMATION FOR SEQ ID NO:33:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:33:
   Ala Ser Phe Leu Ser Thr Ser Ser Ser Leu Glu Tyr
   1               5                   10
(2) INFORMATION FOR SEQ ID NO:34:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:34:
   Ala Ser Tyr Leu Ser Thr Ala Ser Ser Leu Glu Tyr
   1               5                   10
(2) INFORMATION FOR SEQ ID NO:35:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (v) FRAGMENT TYPE: internal
   (xi) SEQUENCE DESCRIPTION: SEQ ID NO:35:
   Val Ser Tyr Leu Ser Thr Ala Ser Ser Leu Asp Asn
   1               5                   10
(2) INFORMATION FOR SEQ ID NO:36:
   (i) SEQUENCE CHARACTERISTICS:
      (A) LENGTH: 321 base pairs
      (B) TYPE: nucleic acid
      (C) STRANDEDNESS: double (D) TOPOLOGY: linear
   (ii) MOLECULE TYPE: cDNA
   (xi) SEQUENCE DESCRIPTION: SEQ ID NO:36:
   GACATCCAGA TGACCCAGTC TCCATCCTCC CTGTCTGCAT CTGTAGGGGA CAGAGTCACC
   60
   ATCACTTGTC GGGCAAGTCA GGGCATCAGA AATTACTTAG CCTGGTATCA GCAAAAACCA
   120
   GGGAAAGCCC CTAAGCTCCT GATCTATGCT GCATCCACTT TGCAATCAGG GGTCCCATCT
   180
   CGGTTCAGTG GCAGTGGATC TGGGACAGAT TTCACTCTCA CCATCAGCAG CCTACAGCCT
   240
   GAAGATGTTG CAACTTATTA CTGTCAAAGG TATAACCGTG CACCGTATAC TTTTGGCCAG
   300
   GGGACCAAGG TGGAAATCAA A
   321
(2) INFORMATION FOR SEQ ID NO 37:
   (i) SEQUENCE CHARACTERISTICS:
      (A) LENGTH: 363 base pairs
      (B) TYPE: nucleic acid
      (C) STRANDEDNESS: double
      (D) TOPOLOGY: linear
(ii) MOLECULE TYPE: cDNA
(xi) SEQUENCE DESCRIPTION: SEQ ID NO:37:
   GAGGTGCAGC TGGTGGAGTC TGGGGGAGGC TTGGTACAGC CCGGCAGGTC CCTGAGACTC
   60
   TCCTGTGCGG CCTCTGGATT CACCTTTGAT GATTATGCCA TGCACTGGGT CCGGCAAGCT
   120
   CCAGGGAAGG GCCTGGAATG GGTCTCAGCT ATCACTTGGA ATAGTGGTCA CATAGACTAT
   180
   GCGGACTCTG TGGAGGGCCG ATTCACCATC TCCAGAGACA ACGCCAAGAA CTCCCTGTAT
   240
   CTGCAAATGA ACAGTCTGAG AGCTGAGGAT ACGGCCGTAT ATTACTGTGC GAAAGTCTCG
   300
   TACCTTAGCA CCGCGTCCTC CCTTGACTAT TGGGGCCAAG GTACCCTGGT CACCGTCTCG
   360
   AGT
   363